# EUROPEAN PATENT APPLICATION

(11) **EP 4 640 056 A1**
(43) Date of publication of application: **29.10.2025**
(21) Application number: 24305646.2
(22) Date of filing: 25.04.2024
(51) Int. Cl.: A01N 63/40

(54) **ISOLATED BACTERIOPHAGE AGAINST XYLELLA FASTIDIOSA**

(71) Applicant: Bioline Agrosciences France, 75016 Paris (FR); Centre National de la Recherche Scientifique (CNRS), 75016 Paris (FR); Université d'Aix Marseille, 13007 Marseille (FR)
(72) Inventor: LEBRUN, Caroline, 31400 TOULOUSE (FR); GRAILLOT, Benoît, 64510 BOEIL-BEZING (FR); GINET, Nicolas, 13008 MARSEILLE (FR); ANSALDI, Mireille, 13001 MARSEILLE (FR)
(74) Representative: Regimbeau

(57) **Abstract**

The invention relates to an isolated bacteriophage that is aimed at *Xylella fastidiosa,* wherein the bacteriophage:
(a) is capable of inhibiting the growth of said *Xylella fastidiosa;*
(b) comprises a flexible non-contractile tail with a length > 220 nm and an icosahedral capsid
(c) exhibits a genomic size of about 55000 bp to 55500 bp; and
(d) comprises a genome with a DNA sequence having at least 92% sequence identity to SEQ ID NO:1.

## Description

### FIELD OF THE INVENTION

The present invention relates to the field of plant pathology and biological control agents. More specifically, the invention relates to methods and compositions for treatment of plant diseases caused by Xylella fastidiosa comprising the use of a bacteriophage, a virus of bacteria.

### BACKGROUND OF THE INVENTION

Bacteria can cause many infectious diseases in plants. The bacteria infect plant tissues and can cause wilting, poor growth, lesions on fruit, and even plant death. Infection can occur through spreading by wind, rain, contaminated equipment, or vector insects, rapidly spreading to other plants, and resulting in deleterious effects to the plant and massive crop losses. Effective treatment of these diseases requires a method of treating the plant to eliminate the bacteria.

*Xylella fastidiosa* (*Xf*) is a slow-growing Gram-negative plant-pathogenic bacterium emerging in Asia and Europe, and is therefore listed as a quarantine organism [1], Long known in the Americas, this xylem-specialized bacterium is associated with numerous socio-economically important plant diseases. The impact of diseases due to *Xylella* include direct and indirect economic damages that were estimated to reach 150 million dollars per year in California when including measures to control vectors [2,3]. In the European olive oil production area, the projected impact of olive quick decline syndrome (OQDS) over 50 years ranges from two to five billion euros [4]. Nonetheless, such diseases have wider impacts; for instance, the impairment was estimated at 30% of the provision of ecosystem services provided by agroecological systems, including landscape value, cultural and natural heritage in the Puglia province of Italy [5]. *Xf* is transmitted by xylem sap-feeding insects and belongs to the *Xanthomonadaceae* (synonymous with the *Lysobacteraceae)* family. Xf genome has been the first plant pathogen genome sequenced, highlighting its importance as a plant pest [6]. Its genome of 2.6 Mb with a low GC content (52.7%) is smaller than that of other *Xanthomonadaceae,* even the ones of the phylogenetically closest relatives *Xanthomonas albilineans (Xa)* and X. *translucens (Xt)* whose genomes are composed of 3.7 and 4.7 Mb, respectively [7,8]. It is tempting to link the Xf reduced genome to its fastidious growth. Interestingly, a recent genome-scale metabolic network reconstruction analysis suggested that the Xf metabolic network is pretty much complete, although minimalist and not particularly robust [9]. Indeed, alternative reactions seem to be missing; the metabolism of the bacteria thus relies on poorly efficient metabolic pathways and lack of flexibility. Moreover, the study also found that the production of some virulence factors, such as exopolysaccharides (EPS), is at a high cost to the plant and is thus detrimental to fast growth [9].

*Xf* has been identified as the causative agent of Pierce's disease, which has been causing extensive damage to vineyards in California for almost 150 years [10]. However, it was only at the end of the 1970s that this fastidious bacterium could be isolated on solid medium [11]. Since then, *Xf* has been associated with various forms of plant diseases on many different plant hosts [12]. Today, this bacterium belongs in the top 10 of the plant pathogens that are most studied worldwide [13]. *Xf* is endemic in the Americas and is persistently involved in large epidemics, causing significant damage in vineyards and citrus orchards. Listed as a quarantine pest in Europe, the three main *Xf* subspecies *(pauca (Xfp), multiplex (Xfm)* and subsp. *fastidiosa* (*Xff*)) were recently detected in Europe, in particular in the Mediterranean area, probably introduced through commercial exchanges and causing a variety of diseases such as the leaf scorch of olive trees in Italy [14]. Continuous monitoring using standardized real-time PCR protocols is now performed in southern Europe [15,16], However, in the absence of any efficient and authorized chemical method to control Xf, a major challenge is to develop environmentally friendly biotechnologies to control this plant disease. The current strategy implemented by the European Commission to prevent Xf spread within the EU consists of eradication and containment measures involving intensive surveillance, vector control treatments, and differential removal of infected plants and specified hosts in foci and buffer zones [17]. Additional measures to these economically and socially devastating ones have been proposed, such as the selection of naturally resistant host plants, the thermal treatment of young plants, or the use of a combination of zinc, copper, manganese and citric acid [18,19,20]. Biocontrol strategies have also been proposed using avirulent Xf strains or other avirulent plant colonizers [21,22]. However, none of these later strategies has been recognized as fully efficient or totally safe by the European authorities.

Bacteriophages, the viruses infecting bacteria, have been used to treat infectious diseases since their discovery at the beginning of the twentieth century [23], Their use in therapy has pros and cons, but the clear advantages are that they can multiply in the presence of a host so there's no need to apply them regularly and can be adapted to any bacterial species as long as strictly virulent phages can be isolated. Recently, the agricultural sciences community put a great deal of emphasis on phage therapy to develop sustainable strategies of biocontrol for plants and animals [24,25,26]. Currently, a number of phage products are being developed or are already commercially available for a variety of plant pathogens such as various *Xanthomonas* strains or *Ralstonia solanacearum* [27]. As an example, the OmniLytics^{™} company commercialized several phage products under the AgriPhage^{™} name to control tomato and pepper bacterial spot and speck caused by *Xanthomonas euvesicatoria* pv. *Euvesicatoria* and *Pseudomonas syringae* pv. *tomato,* as well as other plant diseases. In this context, a group from Texas A&M University proposed the use of bacteriophages as a treatment for Pierce's disease [28,29,30], the product name is XylPhi^{®} https://inphatec.com/xylphi_pd.

Clavijo-Coppens et al. also attempted to isolate Xf-specific phages focusing on the three *Xf* subspecies present in the Mediterranean basin. [31].

There still exists a need in treatments against diseases caused by *Xylella fastidiosa.*

### SUMMARY OF THE INVENTION

The first subject-matter of the invention is an isolated bacteriophage that is aimed at *Xylella fastidiosa,* wherein the bacteriophage:
(a) is capable of inhibiting the growth of said *Xylella fastidiosa;*
(b) comprises a flexible non-contractile tail with a length > 220 nm and an icosahedral capsid
(c) exhibits a genomic size of about 55000 bp to 55500 bp; and
(d) comprises a genome with a DNA sequence having at least 92% sequence identity to SEQ ID NO:1.

The second subject-matter of the invention is a method of preventing or reducing symptoms or disease caused by *Xylella fastidiosa* in a plant, comprising contacting said plant or a part of said plant with at least one bacteriophage according to the invention.

The third subject-matter of the invention is a biocontrol composition comprising at least one bacteriophage according to the invention and a carrier.

### DESCRIPTION OF THE DRAWINGS

**Figure 1****:** Genomic comparison tree created using the dRep tool for all the phages in our library. The abbreviations are as follows: phg = phage, Xant = Xanthomonas, Xyle = Xylella. Published phages are followed by their accession number. The black dotted line is placed at 70% identity.
**Figure 2****:** Genomic comparison tree created using the dRep tool for all the phages in our library. The abbreviations are as follows: phg = phage, Xant = Xanthomonas, Xyle = Xylella. Published phages are followed by their accession number. The dotted black line is placed at 70% identity. Shades of grey correspond to the VCs formed by vConTACT2: VC_1, VC_2_1, VC_3, VC_24, VC_58, VC_135.
**Figure 3****:** GBDP phylogenomic tree on the amino acid sequence of VC_24 phages produced by VICTOR using formula D6. The numbers above the branches are the GBDP pseudo-bootstrap support values from 100 replications. The branch lengths of the resulting VICTOR trees are scaled according to the respective distance formula used. G = genus.
**Figure 4****:** VIRIDIC heatmap of VC_24 phages. Abbreviations: phg = phage, Sten = Stenotrophomonas, Xant = Xanthomonas, Xyle = Xylella. Genera as currently described in the ICTV are added.
**Figure 5****:** Transmission Electron Microscope images of newly isolated phages. Error bar = 200 nm. A. Learch, B. Karst, C. Horst, D. Caracole, E. SERU 7970 2.1, F. SERU 7970 3.1, G. SERU 7970 4.1, H. Sov, I. SERU 7970 9a1.1, J. SERU 7970 9b1.1, K. SERU 7970 10.1, L. SERU 7970 11. 1, M. SERU 7970 12.1, N. SERU 7970 13.1, O. SERU 7970 14.1, P. Steppe, Q. Oroshi, R. SERU 8402 2.1, S. Darbon, T. Arval, U. Firost, V. Larco, W. Tourse, X. Boscavo, Y. Aoi, Z. Alme.
**Figure 6****:** Measurements of phage tail lengths in ascending order in nm. Statistical groups according to the Bonferroni (bon) or Holm (holm) fit and statistical groups resulting from the analysis of these two fits (capitals).
**Figure 7****:** Superposition of statistical groups of siphovirus tail lengths on the genomic comparison tree.
**Figure 8****:** Overlays optimization of Xf multiplex 8416. A, Without overlay, Xf aggregates. B, after the optimization of all parameters. All phages tested here provoke Xf growth inhibition, unlike the negative control (buffer spot).
**Figure 9****:** Growth of Xylella fastidiosa pauca 8402 with an initial OD₆₀₀ of 0.1, 0.05, 0.01 without phages (circles), with 10⁵ PFU/mL Bacata (triangles), and 10⁸ PFU/mL Bacata (squares).

### DESCRIPTION OF THE SEQUENCE LISTING

SEQ ID NO:1 = Aoi VP2 7970 2.1 genomic sequence
SEQ ID NO:2 = Oroshi SERU 8402 1.1 genomic sequence
SEQ ID NO:3 =Alme VP2 8418 1.1 genomic sequence
SEQ ID NO:4 = primer Cota F
SEQ ID NO:5 = primer Cota R
SEQ ID NO:6 = primer Usme F
SEQ ID NO:7 = primer Usme R
SEQ ID NO:8 = primer Bacata F
SEQ ID NO:9 = primer Bacata R
SEQ ID NO:10 = primer Bosa F
SEQ ID NO:11 = primer Bosa R
SEQ ID NO:12 = primer Aoi F
SEQ ID NO:13 = primer Aoi R
SEQ ID NO:14 = primer Alme F
SEQ ID NO:15 = primer Alme R
SEQ ID NO:16 = primer Horst F
SEQ ID NO:17 = primer Horst R
SEQ ID NO:18 = primer Learch F
SEQ ID NO:19 = primer Learch R
SEQ ID NO:20 = primer Oroshi F
SEQ ID NO:21 = primer Oroshi R
SEQ ID NO:22 = primer Darbon F
SEQ ID NO:23 = primer Darbon R
SEQ ID NO:24 = primerArval F
SEQ ID NO:25 = primerArval R
SEQ ID NO:26 = primer Caracole F
SEQ ID NO:27 = primer Caracole R
SEQ ID NO:28 = primer Sov F
SEQ ID NO:29 = primer Sov R
SEQ ID NO:30 = primer Firost F
SEQ ID NO:31 = primer Firost R
SEQ ID NO:32 = primer Larco F
SEQ ID NO:33 = primer Larco R
SEQ ID NO:34 = primer Tourse F
SEQ ID NO:35 = primer Tourse R
SEQ ID NO:36 = primer Boscavo F
SEQ ID NO:37 = primer Boscavo R

| Bacteriophage Name | DSMZ number | DSMZ date of deposit | Genomic sequence |
|---|---|---|---|
| Aoi VP2 7970 2.1 | 34782 | September 26, 2023 | SEQ ID NO:1 |
| Oroshi SERU 8402 1.1 | 34799 | October 11, 2023 | SEQ ID NO:2 |
| Alme VP2 8418 1.1 | 34795 | October 11, 2023 | SEQ ID NO:3 |

DSMZ is an international depositary authority under the Budapest treaty (Leibniz Institute DSMZ-German collection of Microorganisms and cell Cultures, Inhoffenstrasse 7B, D-38124 Braunschweig).

### DEFINITIONS

Throughout this document, values expressed in a range format should be interpreted in a flexible manner to include not only the numerical values explicitly recited as the limits of the range, but also to include all the individual numerical values or sub-ranges encompassed within that range as if each numerical value and sub-range is explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise.

In this document, the terms "a", "an" or "the" are used to include one or more than one unless the context clearly dictates otherwise.

A bacteriophage, also referred to as a phage, is a virus that infects and replicates within bacteria. Bacteriophages include proteins that encapsidate a DNA or RNA genome as well as all the equipment required for genome injection, and may have structures that are either simple or elaborate. Their genomes may encode as few as four genes and as many as hundreds of genes. Phages replicate within the bacterium following the injection of their genome into its cytoplasm.

Bacteriophages are obligate intracellular parasites that multiply inside bacteria by co-opting the host biosynthetic machineries. Phages contain nucleic acid and protein, and may be enveloped by a lipid membrane. Depending upon the phage, the nucleic acid genome can be either DNA or RNA but not both, and can exist in either circular or linear forms, single- or double-stranded. The size of the phage genome varies depending upon the phage. The simplest phages have genomes that are only a few thousand nucleotides in size, while the more complex phages may contain more than 100,000 nucleotides in their genome, and in rare instances more than 1,000,000. The number and amount of individual types of protein in phage particles will vary depending upon the phage. Some of the proteins play a role in infection and to protect the nucleic acid genome from environmental nucleases.

Phage genomes come in a variety of sizes and shapes (e.g., linear or circular). Most phages range in size from 24-200 nm in diameter. The capsid is composed of many copies of one or more phage proteins, and acts as a protective envelope around the phage genome. Many phages have tails attached to the phage capsid. The tail is a hollow tube through which the phage nucleic acid is ejected during infection. The size of the tail can vary and some phages do not even have a tail structure. In the more complex phages, the tail is surrounded by a contractile sheath which contracts during infection of the bacterial host cell. At the end of the tail, phages have a baseplate and one or more tail fibers attached to it. The baseplate and tail fibers are involved in the binding of the phage to the host cell's receptor.

An "isolated" molecule is one which has been identified and separated and/or recovered from a component of its natural environment.

As used herein, the term "virulent" refers to a virus, particularly a bacteriophage, that replicate exclusively through a lytic cycle, and thus is able to infect, replicate within, and lyse (kill) a host cell.

The term "temperate" refers to a bacteriophage that, alternatively to a lytic development, can undergo lysogeny, and commonly able to integrate into the host genome (lysogenize).

Phages are propagated in an appropriated host, as is described herein. The term "host" refers to a bacterial cell that is sensitive to bacteriophage. The bacterial host may be a non-natural, for example genetically modified, bacterial host cell or a natural bacterial host cell.

The percent identities referred to in the context of the disclosure of the present invention are determined after optimal alignment of the sequences to be compared, which may therefore comprise one or more insertions, deletions, truncations and/or substitutions.

This percent identity may be calculated by any sequence analysis method well-known to the person skilled in the art.

The percent identity may be determined after global alignment of the sequences to be compared of the sequences taken in their entirety over their entire length. In addition to manual comparison, it is possible to determine global alignment using the algorithm of Needleman and Wunsch (1970).

For nucleotide sequences, the sequence comparison may be performed using any software well-known to a person skilled in the art, such as the Needle software. The parameters used may notably be the following: "Gap open" equal to 10.0, "Gap extend" equal to 0.5, and the EDNAFULL matrix (NCBI EMBOSS Version NUC4.4).

For amino acid sequences, the sequence comparison may be performed using any software well-known to a person skilled in the art, such as the Needle software. The parameters used may notably be the following: "Gap open" equal to 10.0, "Gap extend" equal to 0.5, and the BLOSUM62 matrix.

Preferably, the percent identity as defined in the context of the present invention is determined via the global alignment of sequences compared over their entire length.

As used herein, the terms "treatment," "treating," and "treat" are defined as acting upon a disease, disorder, or condition with an agent to reduce or ameliorate the physiologic effects of the disease, disorder, or condition and/or its symptoms.

"Treatment," as used herein, covers any treatment of a disease in a plant host {e.g., a plant species, including those of agricultural interest, such as edible plants or those used to produce edible products, as well as ornamental plant species), and includes: (a) reducing the risk of occurrence of the disease in a plant, (b) impeding the development of the disease, and (c) relieving the disease, i.e., causing regression of the disease and/or relieving one or more disease symptoms. "Treatment" is also meant to encompass delivery of an inhibiting agent to provide an effect, even in the absence of a disease or condition. For example, "treatment" encompasses delivery of a disease or pathogen inhibiting agent that provides for enhanced or desirable effects in the plant (e.g., reduction of pathogen load, reduction of disease symptoms, etc.).

The term "carrier" refers to a diluent, adjuvant, surfactant, excipient, or vehicle with which the phage is administered. Such carriers can be sterile liquids, such as water and oils, including those of petroleum, animal, vegetable or synthetic origin, such as peanut oil, soybean oil, mineral oil, sesame oil and the like. Saline solutions, including phosphate solution such as sodium monohydrogen phosphate, potassium dihydrogen phosphate and aqueous dextrose and glycerol solutions can also be employed as liquid carriers, particularly for injectable solutions. Suitable excipients may include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like.

The term *"Siphoviruses"* means a morphology of bacteriophages with a long flexible and noncontractile tail [52].

"Inhibiting the growth" means reducing or even halting the growth rate or even killing the bacteria. In particular, this means reducing the growth of biofilm or reducing biofilm formation or preventing biofilm formation.

### DETAILED DESCRIPTION OF THE INVENTION

### Bacteriophages

The first subject-matter of the invention is an isolated bacteriophage that is aimed at Xylella fastidiosa (*Xf*), wherein the bacteriophage :
(a) is capable of inhibiting the growth of said *Xylella fastidiosa;*
(b) comprises a flexible non-contractile tail with a length > 220 nm and an icosahedral capsid;
(c) exhibits a genomic size of about 55000 bp to 55500 bp; and
(d) comprises a genome with a DNA sequence having at least 92% sequence identity to SEQ ID NO:1.

Preferably, the bacteriophage according to the invention has a siphovirus morphology, i.e. possess a long flexible and non-contractile tail.

Preferably, the isolated bacteriophage according to the invention comprises a genome with a DNA sequence having at least 93% sequence identity to SEQ ID NO:1, more preferably 93%, even more preferably 94%.

Advantageously, the isolated bacteriophage according to the invention comprises a genome with a DNA sequence selected in the group consisting of SEQ ID NO:1, SEQ ID NO:2 and SEQ ID NO:3.

Preferably, the isolated bacteriophage according to the invention is selected in the group consisting of the bacteriophages Aoi, Oroshi and Alme deposited respectively under the following DSMZ Accession Numbers : DSM 34782, DSM 34799 and DSM 34795.

### Sub-species

Preferably, the isolated bacteriophage according to the invention is virulent to one of the three main *Xf* subspecies *(pauca (Xfp), multiplex (Xfm)* and subsp. *fastidiosa* (*Xff*))*.*

More preferably, the isolated bacteriophage according to the invention is virulent to two of the three main *Xf* subspecies *(pauca (Xfp), multiplex (Xfm)* and subsp. *fastidiosa* (*Xff*))*.*

Advantageously, the isolated bacteriophage according to the invention is virulent to the three main *Xf* subspecies *(pauca (Xfp), multiplex (Xfm)* and subsp. *fastidiosa* (*Xff*))*.*

### Plant diseases

According to the host plants, *Xf* induced diseases are revealed by a large variety of symptoms that are diverse and non-specific (can be similar to dryness effects); symptoms can be evenly or unevenly distributed throughout the plant, affecting leave development and fruit production. It's been recognized that *Xf* capacity to form biofilms in the plant vessels alters water and nutrient transportation. In the most serious cases, the disease can lead to the death of the plant through branch desiccation and drying. Depending on the plant, the following symptoms can be observed:
- lack of shoots and persistent petioles
- sectorial drying of the leaf blade
- leaf chlorosis
- yellowing or reddening of leaves.

Notably, for some host plants, *Xf* infection is asymptomatic and those infections may function as *Xf* natural reservoirs.

### Plants

Plant species able to be infected by Xylella are listed, for example, at Annex I and Annex II of the Commission Implementing Regulation (EU) 2020/1201 of 14 August 2020 as regards measures to prevent the introduction into and the spread within the Union of Xylella fastidiosa (Wells et al.) (as amended on September 28, 2023) (http://data.europa.eu/eli/reg_impl/2020/1201/2023-09-28) and may include commercial crops.

In the Commission Implementing Regulation (EU) 2020/1201:
(a) 'specified pest' means *Xylella fastidiosa* (Wells *et al.)* and any of its subspecies;
(b) 'host plants' means all plants for planting, other than seeds, belonging to the genera or species listed in Annex I;
(c) 'specified plants' means host plants for planting, other than seeds, belonging to the genera or species listed in Annex II and known to be susceptible to the specific subspecies of the specified pest.

Preferably, the plant likely to be infected by *Xf* is a 'host plant' listed in Annex I or a 'specified plant' listed in Annex II of the Commission Implementing Regulation (EU) 2020/1201, more preferably a plant likely to be infected by *Xf* is a 'host plant' listed in Annex I.

Preferably, the plant likely to be infected by *Xf* is a grapevine, olive tree, citrus tree, almond tree, coffee tree, blackberry tree, mulberry tree, maple tree, walnut tree, date tree, pistachio tree, plane tree, plum tree, pear tree or oak tree.

### Method

The second subject-matter of the invention is a method of preventing or reducing symptoms or disease caused by *Xylella fastidiosa* in a plant, comprising contacting said plant or a part of said plant with at least one bacteriophage according to the invention.

Preferably, contacting comprises introducing bacteriophage particles into the plant.

In another embodiment, contacting comprises seed treatment.

Preferably, contacting comprises introducing bacteriophage into the plant by injection, by plant puncturing, by an insect vector, via the root system by soil-based or watering system delivery, by spray, by mist, or by dust contacting the plant.

Preferably, the number of said bacteriophage(s) introduced into said plant is from 1.10⁵ to 1.10¹⁰ PFU/ml.

Preferably, at least two bacteriophages according to the invention are simultaneously or sequentially used.

### Biocontrol composition

The third subject-matter of the invention is a biocontrol composition comprising at least one bacteriophage according to the invention and a carrier.

Preferably, the biocontrol composition according to the invention comprises at least two bacteriophages according to the invention.

Depending growing age of tree, the thickness of the stem, the size of the root, the dosage is adjusted appropriately. A plant disease biocontrol composition can be a dry product, a substantially dry product, a liquid product, or a substantially liquid product. In some embodiments, a dry or substantially dry product can be reconstituted in a liquid (e.g., water, etc.), and then applied to a plant. In other embodiments, such a composition can be applied in dry or substantially dry form, where liquid that is already present on the plant, is concurrently applied to the plant, or that subsequently appears on the plant (e.g., by application, condensation, etc.) facilitates exposure of the bacteriophage to target bacteria. In another embodiment, such a composition can be applied by spray, mist, or dust on the plant.

A plant disease biocontrol composition can take the form of a solution, a suspension, an emulsion, a powder, a tablet, and the like.

The timing of application of a plant disease biocontrol composition is not limited, but may for instance be daily, weekly, or twice-weekly, monthly, bimonthly, or quarterly.

The bacteriophage composition can include a bacteriophage component in a range of from 1.10⁴ to 1.10¹² plaque-forming units/g ("PFU/mL") of the bacteriophage composition, about 1.10⁶ to 1.10¹⁰ PFU/mL, about 1.10⁶ to 5.10⁸ PFU/mL, less than, equal to, or greater than about 1.10⁴ PFU/mL, 1.10⁵, 1.10⁶, 1.10⁷, 1.10⁸, 1.10⁹, or about 1.10¹⁰ PFU/mL.

### EXAMPLES

### MATERIAL AND METHODS

### Bacterial strain and Media

Bacterial strains and culture conditions were mainly taken from the work [31]. Bacterial strains used in this study are listed in Table 1 and were provided by the French Collection of Plant-associated Bacteria (CIRM-CFBP, https://www6.inrae.fr/cirm_eng/ CFBP-Plant-Associated-Bacteria). *Xylella fastidiosa* (*Xf*) strains were cultured on PD3 prepared by substituting soluble potato starch (Sigma) at 2 g.l⁻¹ for the BSA in the PD2 medium, thus making the medium completely autoclavable [33]. The originally described PD2 media contains Tryptone 4.0 g.l⁻¹, Soytone 2.0 g.l⁻¹, trisodium citrate 1.0 g.l⁻¹, disodium succinate 1.0 g.l⁻¹, hemin chloride 0.01 g.l⁻¹, MgSO₄·7H₂O 1.0 g.l⁻¹, KH₂PO₄ 1.0 g.l⁻¹, K₂HPO₄ 1.5 g.l⁻¹, and bovine serum albumin fraction five 2.0 g.l⁻¹. For solid medium, Bacto-agar 12.0 g.l⁻¹ was added to PD3 broth. Cultures on solid medium were incubated at 28°C up to seven days. Liquid cultures of *Xf* strains were incubated at 28°C under 160 rpm agitation. *Xanthomonas* strains were cultured on YPG (yeast extract 7 g.l⁻¹, peptone 7 g.l⁻¹, glucose 7 g.l⁻¹, pH 7.0 to 7.2) [35]. For solid medium, 15 g.l-1 agar was added to YPG broth. Cultures on solid medium were incubated for up to 48 h at 28 °C. For liquid cultures, Xanthomonas strains were incubated at 28 _{°}C under 160 rpm agitation. For *Xanthomonas albilineans (Xa)* overlays, YPG soft agar medium (YPG broth with agar 7.5 g.l⁻¹ added) was used and plates incubated at 28 _{°}C for up to three days. *ForXf strains* storage, cellular suspensions made from fresh cultures were stored at -80 _{°}C in YP glycerol medium (yeast extract 5 g.l⁻¹, peptone 5 g.l⁻¹, glycerol 30% v/v). ForXa storage, the cellular suspensions were stored directly in sterile distilled water at -80°C.

**Table 1: Strains used in this study**

| **Strain/Genotype** | **Abbreviation** | **Collection Code** | **NCBI** |
|---|---|---|---|
| Xylella fastidiosa subsp. fastidiosa | Xff | CFBP 7970 | PRJNA417585 |
| Xylella fastidiosa subsp. pauca | Xfp | CFBP 8402 | PRJNA383475 |
| Xylella fastidiosa subsp. multiplex | Xfm | CFBP 8416 | PRJNA314983 |
| Xylella fastidiosa subsp. multiplex | Xfm | CFBP 8418 | PRJNA314986 |
| Xanthomonas albilineans | Xa | CFBP 2523 | PRJNA338244 |

### Environmental samples for phage isolation

Environmental samples originate from the study of [31]. Briefly, sewage effluents were collected from the wastewater treatment plant of Marseille Provence Metropole (sampling authorized by Marseille Provence Metropole); post-rain runoff waters were collected from Marseille Vieux-Port, France (GPS coordinates 43.2941456, 5.373712). Samples were filtered through a 0.22 µm pore-size nylon syringe filters and then stored at 4 °C. Phages were then pelleted by high-speed centrifugation at 90,000 g for one hour at 4 °C. The pellet was resuspended in 10 mL of phage buffer (PB: 100 mM Tris-HCl pH 7.5, 100 mM NaCl, 10 mM MgCl₂, 10 mM MgSO₄) then stored at 4 °C.

### Phage enrichment

Protocols of document [31] were followed for phage enrichments with PD2 medium replaced by PD3. To select directly forXf-specific phages, enrichments from the environmental samples were conducted on *Xf* cultures. *Xf* strains were first grown at 28 °C on solid medium for up to 15 days. The bacterial lawn was harvested and transferred to 2 mL PD3 broth medium. Then 200 µL of pre-treated environmental samples were added to the cellular suspensions. The cultures were incubated for 10 days at room temperature (RT) without agitation. Xf cells were then removed by centrifugation (4,000 g for 15 min) and the supernatants containing phage particles were filtered through a 0.22 µm pore-size Acrodisc^{®} filter. Enrichment products were then stored in sterile conditions at 4 °C.

### Phage isolation, propagation, purification and titration

The double agar overlay technique [38] on the surrogate host *Xa* was used to isolate phages from the enriched supernatants. Briefly, Xa was cultured overnight in YPG broth at 28°C under 160 rpm agitation. Then 200 µL of bacterial culture were mixed with 5 mL of YPG soft agar kept liquid at 50 _{°}C and 10 µL of serial 10-fold dilutions of the enrichment products were added. The mixture was vortexed and plated on YPG agar plates and plates were incubated for two to three days at 28 °C. Phage lysis plaques were selected and plugged off from the agar surface bottom using a 1000 µL tip and then resuspended in 200 µL of PB. Tubes were vortexed and incubated under 160 rpm agitation for at least 2 h. Cells were lysed by the addition of 5 µL of chloroform (vortexed for 15 s and incubated for 5 min at room temperature). A centrifugation step (10,000 g, 10 min) was done to pellet cell debris and chloroform. The aqueous supernatant (phage lysate) was then recovered and stored at 4°C. Re-isolation was performed on Xa overlay at least three times, repeating plugging and resuspension steps to select single plaques with a regular shape.

Phage particles were then propagated by adding 100 µL of phage suspension to a 6 mL Xa liquid culture in YPG broth at an Optical Density at 600 nm (OD₆₀₀) of 0.1. This mix was incubated at 28 °C under 160 rpm shaking during 48 h, then cells were removed by centrifugation at 5000 g for 15 min and supernatants were filtered through a 0.22 µm pore size Acrodisc^{®} syringe filter.

For phage purification, phage particles were pelleted three times (20,800 g, 1 hour at 4°C). Between each centrifugation, the phage pellets were resuspended in PB. Final phage suspensions were filtered through a 0.22 µm filter and stored at 4 °C.

Phage titration by the double agar overlay plaque assay was performed according to [38]. Phage titers are expressed in Plaque Forming Unit per mL (PFU/mL).

### PCR

In order to reduce the number of isolated phages by eliminating phages too similar to previously published ones, a selection was operated with specifics primers designed by [31]. Thus, whenever a PCR product was detected, the considered phage was excluded from downstream analyses. The remaining phages were considered as potential new phages. A second PCR screening of these remaining phages was performed after genome sequencing a first batch of phage that allowed to design new sets of specific primers.

### Specific primers are the following:

**Table 2: Specific primers used for the pre-selection of isolated phages in bold type, other specific primers of our collection. Alme primers also amplify Oroshi, and Oroshi primers also amplify Alme and Aoi**

| **Name** | **Sequence (5'->3')** | **Tm °C** | **Amplicon size** |
|---|---|---|---|
| **Cota F** | GCGCAGTCATGAGATCGAGA | 53.9 | 307 |
| **Cota R** | GTTGAGTGCACCGAGAGTGA | | |
| **Usme F** | TGGGTCACTTCGGTGTTACG | 53.7 | 320 |
| **Usme R** | GTGGTGAAAGGTGCGGTTTC | | |
| **Bacata F** | CGTAGTCGCGATTGTCGGTA | 53 | 340 |
| **Bacata R** | ACCACTGCTGACGTTCGATT | | |
| **Bosa F** | GCTACCGGTACGACTACGTG | 57 | 362 |
| **Bosa R** | GATCTGGAAACAGCCGTCCA | | |
| Aoi F | TTTCGGTGGCTTGGATTTT | 55.6 | 301 |
| Aoi R | ATTTGCGAGAAGTACAACTG | 54.0 | |
| Alme F | ATCCCCTTTGCATCTTCAAT | 55.9 | 324 |
| Alme R | TTCCGTCATCTAAGGGAATC | 54.2 | |
| **Horst F** | TAAGCCCTTCGTAAAGTTCT | 54.0 | 413 |
| **Horst R** | AAGCATTGAAAAACGAAACG | 54.0 | |
| **Learch F** | ACTAGGAGAAGATTAGATGGC | 53.9 | 448 |
| **Learch R** | CTGGTCCGTAAGGGTG | 52.8 | |
| **Oroshi F** | GATATACGAGCAACGTCTTC | 53.8 | 493 |
| **Oroshi R** | ATTTGCGAGAAGTACAACTG | 54.0 | |
| **Darbon F** | GTTATTGCGACGGTTAAAGA | 54.1 | 442 |
| **Darbon R** | TGGATTAAATACTACGCCGA | 54.0 | |
| **Arval F** | CTGGCAAAACAACCTCATTA | 54.1 | 394 |
| **Arval R** | AGTTACCACGAGGATTTCAT | 54.1 | |
| Caracole F | GTAGATGTTCGGTTCGGTA | 54.1 | 333 |
| Caracole R | CAAGCCAATTCACATCGTAG | 54.8 | |
| Sov F | ATTCTGTTTCAACAACCCTG | 54.0 | 456 |
| Sov R | CAGACATCCAGGAACTTTTC | 53.9 | |
| Firost F | CATACCTACGATATACGGCA | 54.0 | 300 |
| Firost R | GACTACTCGCTCTACATCAA | 54.1 | |
| Larco F | ATGGCCTTTCTTCATTCTTG | 53.8 | 448 |
| Larco R | AGAAAATATCCGGGTGTGAT | 54.1 | |
| Tourse F | TAGATTGTGATTCACGCAAC | 54.1 | 345 |
| Tourse R | GAAGACGAAAAGGCAAAAAC | 54.0 | |
| Boscavo F | ATATTGAACTCCGAAGTCGT | 54.2 | 333 |
| Boscavo R | CTACCAAACTGGGGGATAG | 53.9 | |

### Phage host range

According to the clustering bioinformatic analysis, additional bacterial strains were tested for their sensitivity to the phage collection. Strain names and culture conditions are given in the table below:

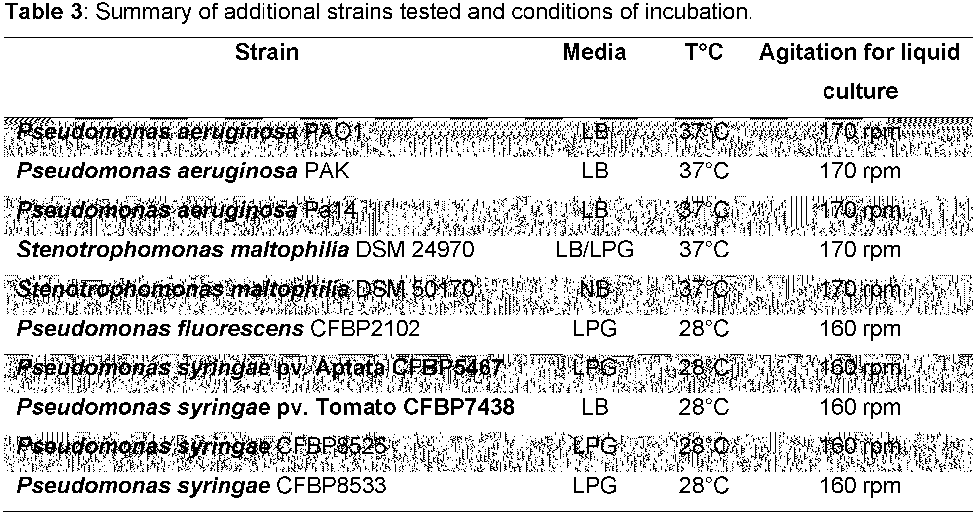

Bacteria were grown on plates (LB, LPG or NB) for one day at 37°C for P. *aeruginosa* and *Stenotrophomonas* strains, and at 28°C for P. *syringae* and P. *fluorescens* strains. Liquid precultures were made O/N from individual colonies and left to grow under the conditions indicated in Table 3.

Culture was started at OD₆₀₀ of 0.05 in 5 mL of medium and left to incubate at the same condition as the preculture until a minimal OD600₆₀₀ of 1.5 was reached.

Media and incubation conditions were adapted to the used strains. Xa overlays were performed in parallel as positive controls.

### Efficiency tests: liquid, biofilm, overlays

### Overlay assays

Several parameters were tested to develop the overlay method with Xf strains:
- media: PWG-M ([51]) and PD3
- final bacterial OD₆₀₀ nm: 0.1, 0.25, 0.5
- percentage of agar in the soft layer: 0.5 and 0.75 %
- soft agar thickness: 3, 5 and 7 mL
- without overlay

After optimization, the overlay assays were performed as follows: *X. fastidiosa* strains *Xff, Xfp* and *Xfm* were cultured at 28 °C on PD3 agar plates up to 7 days. Then, the cultures were suspended into PD3 and the OD₆₀₀ was measured. The volume of *Xf* needed to achieve a final OD₆₀₀ of 0.25 was calculated for 7 mL of 0.75% PD3 soft agar poured over a layer of PD3 agar and left to dry. Then, a 15 µL volume of phages at maximum concentration was deposited on this overlay and left to dry. As negative control 15 µl of phage buffer were also spotted on top of each top-agar culture. Spots were dried at room temperature and the plates incubated up to 10 days at 28 °C.

### Kinetics in liquid media

### Proof of concept

Several parameters were tested to optimize at first the growth of Xf strains in liquid culture and then the infection by phages:
- Initial OD₆₀₀: 0.01, 0.05, 0.1, 0.2, 0.3, 0.4, 0.5
- With or without shaking at 160 rpm
- Initial titre of phages: 10⁵, 10⁷, 10⁸, 10⁹, 10¹⁰ PFU/mL
- Phages: Bacata, Learch, Aoi, Arval, Oroshi, (Xylphi-PDTM-PD^{™} https://inphatec.com/Xylphi-PDTM_pd)

The strains were cultured on PD3 medium and then resuspended in PD3. OD₆₀₀ of these cultures were measured in order to inoculate 4 mL liquid medium at the desired OD₆₀₀ into glass tubes. Incubation was carried out at 28°C, and phages were added 24 hours *afterXylella* inoculation. Growth was monitored for at least 10 days through OD₆₀₀ measurements.

### Culture assays in 96-well plates

*Xylella* strains were cultured on PWG agar plate for 7 days, then cultured again during 7 days. Liquid assays were performed in 96-well ELISA plates. *Xylella* strains were diluted to reach an OD₆₀₀ of 0.1, phages were added at T0 at a final concentration of 10¹⁰ PFU/mL. Culture plates were incubated at 28°C under 200 rpm agitation and measurements were made every 24 h for 7 days. The late addition of phages was also tested (D4 and D7 after bacterial inoculation).

### Biofilm assays

All biofilm assays were carried out in parallel with a kinetics test in 96-well plates. The conditions were therefore identical to those described above except that the plate material was propylene and the absence of agitation during incubation. At the end of the 7-day incubation period, supernatants were removed, and wells were gently rinse twice with 200 µL of deionized water. Then, 200 µL of 0.1% crystal violet was added to each well and allowed to incubate for 15 min at room temperature under static conditions. Cristal violet was removed and gently rinsed with 200 µL of deionized water twice. Labelled exopolysaccharides were solubilized with 200 µL of 100% alcohol and transferred into ELISA plates. Plates were then read on a spectrophotometer using KC4 software (Bio-Tek, Instruments, Winooski, Vermont, USA).

### Negative Staining and Transmission Electron Microscopy and Phage morphological characterization

To obtain the sample to be analyzed under the microscope, a phage isolation step was performed from an overlay of Xa (as described in isolation step). Up to a dozen of phage-forming lysis plaques were plugged off from the agar surface and resuspend in 2 mL of PB. After vortexing and incubation 2 hours under agitation 160 rpm, 20 µL of chloroform was added, homogenized, and remove by 10 min centrifugation at 10000 rpm. 1900 µL of supernatant was filtered through 0.22 µm pore size syringe filter. The phage lysate was stored at 4 °C. Compared to an aliquot of phage liquid propagation, the lysis plaques method allows to limit cell debris which allows a much better image quality and to check the homogeneity of lysis plaques before microscopy. However, the concentration is more difficult to estimate and requires a titration step to be accurate.

2 mL of phage preparations were pelleted by a centrifugation step (1 hour, 20,800× g, 4 _{°}C). Pelleted phages were washed with 2 mL of TEM-buffer (0.22 µm filtered 0.1 M NH4-acetate (pH 7)), pelleted again three times and concentrated in a final volume of 20 µL TEM-buffer. Formvar/carbon-coated copper grids were prepared at the Institut de Microbiologie de la Méditerranée (IMM) Microscopy facility. Shortly, 4 µL drops of diluted phage suspensions were spotted on glow discharged carbon-coated grids (EMS) and let stand for 3 min. The grids were then washed with two drops of 2% aqueous uranyl acetate and stained with a third drop for 2 min. Grids were dried on filter paper. Observations were made on an FEI Tecnai G2 20 TWIN (200KV), laB6, Gatan Oneview 4k × 4k CMOS transmission electron microscope and digital acquisitions were made with a numeric camera (Eagle, FEI). Tail and capsid lengths were obtained by measurements via imaged software [46]. Statistics were performed on R in order to compare phages length between them. The conditions for performing an ANOVA were verified and invalidated, the quantitative variable does not follow a normal distribution for all the populations compared (Shapiro-Wilk test p-value < 0.1 therefore rejection of the hypothesis H0: the sample follows a normal distribution). The nonparametric Kruskall-Wallis test followed by the nonparametric Wilcoxon post hoc test were performed using the Bonferroni and Holm correction for p-value adjustment. Plots are made with R.

### Phage DNA purification

Total phage DNA was extracted according to Gendre et al. [36]. Briefly, the non-encapsidated nucleic acids of 200 µL of purified phages in PB were removed by the addition to the purified phage suspension of 20 µL of DNAse I (6 mg/mL, EUROMEDEX), 2 µL of RNAse A (4 mg/mL, Promega) and 4 µL of Dpnl restriction enzyme (20,000 U/mL, New England Biolabs). The sample was incubated for 1 h at 37 °C then for 20 min at 65 _{°}C under shaking to inactivate the DNAse I. To release Phage DNA, the capsid was damaged by the addition of 20 µL of 10% SDS and 20 µL of proteinase K (50 µg/mL, EPICENTRE) and heating at 56 °C for one hour. The sample volume was completed with PB buffer q.s. 600 µL. Phage DNA was extracted by adding 600 µL phenol/chloroform/isoamyl alcohol (25:24:1, Sigma-Aldrich) and then vortexing the sample for 30 s. After centrifugation (10,000 g, 10 min, 4 °C), the upper aqueous phase containing the nucleic acids was recovered. This step of extraction was repeated twice. The final aqueous phase-containing DNA was then ethanol-precipitated and finally resuspended in 50 µL DNAse/RNase-free water for storage at -20 _{°}C. Phage DNA was quantified with a Nanodrop spectrophotometer.

### Phage genome sequencing

DNA sequencing was realized at the IMM Transcriptomic and Genomic facility following the protocol of Gendre et al. [36]. Phage dsDNA was first quantified with a Qubit^{™} fluorometer in combination with the Qubit^{™} dsDNA HS Assay kit (Invitrogen). Around 750 ng of purified phage genomic DNA was then mechanically fragmented in 50 µL microtubes using a Covaris M220 sonifier with the following parameters: peak power 75W, duty factor 10%, Cycles/Burst 200. Fragmentation (size and distribution profile) was assessed with a Tapestation 4200 System (Agilent) in combination with the D5000 DNA Screen Tape System (Agilent). DNA libraries for high throughput sequencing were prepared from fragmented DNA with the NEBNext^{®} Ultra^{™} II DNA Library Prep kit for Illumina^{®} (Biolabs) according to the manufacturer's protocol. DNA libraries (size and distribution profile) were assessed with a Tapestation 4200 System (Agilent) in combination with the D5000 DNA Screen Tape System (Agilent). DNA libraries were then diluted at 4 nM in the appropriate buffer. Paired-end (2 × 150 bp) DNA sequencing was performed on the MiSeq sequencer (Illumina^{®}, San Diego, CA, USA) with a MiSeq v2 (300-cycles) flow cell (Illumina^{®}, San Diego, CA, USA) according to the manufacturer's protocol.

### Genome clustering

For genome clustering, vContact2 0.9.19 (with the ProkaryoticViralRefSeq201 database accessed on 26 October 2022), a taxonomy classification tool that performs network-based clustering of viral sequences and specifically designed to work on metagenomics data was used. vConTACT2 uses whole-genome gene-sharing profiles between a reference database of curated and annotated bacteriophages and the user's viral sequences to build networks for phage taxonomy [45]. Genomes are grouped based on protein content and similarity, and the resulting clusters are largely concordant with the International Committee on Taxonomy of Viruses (ICTV) viral taxa [32]. vConTACT2 compares the protein sequences of the studied phages to the BLASTp virus database. Protein clustering is performed by the MCL (Markov cluster) tool built on the basis of shared homologous proteins. ClusterONE then performs the clustering of the viral genomes based on the MCL results. The clustering scores (PC or VC) were calculated by Vcontact2.

### Taxonomic classification

Virus Classification and Tree Building Online Resource (VICTOR) was used to better understand the phylogenetic relationships between phages of the same VC with the amino acid option. When relevant, the VICTOR analysis is expanded with phages close to those contained in the VC in the ICTV taxonomic classification. Phage sequences were obtained from the NCBI nucleotide database. This tool is available online: https://ggdc.dsmz.de/victor.php (accessed on 15 November 2022). VICTOR is a method for the genome-based phylogeny and classification of prokaryotic viruses [41]. It performs all pairwise comparisons of the protein sequences of the phages studied using the Genome-BLAST Distance Phylogeny (GBDP) method [40] under settings recommended for prokaryotic viruses [41]. All distances are calculated from matches (local alignments) between two proteome sequences. These matches are known as HSPs (high-scoring segment pairs). The branch lengths of the resulting VICTOR trees are scaled in terms of these distance formulas: d4 is the sum of all identities found in HSPs divided by overall HSP length (correspond to Genome to Genome Distance Calculator (GGDC) formula 2) and d6 is the sum of all identities found in HSPs divided by total genome length (correspond to GGDC formula 3). The resulting intergenomic distances were used to infer a balanced minimum evolution tree with branch support via FASTME including SPR postprocessing [39] for each formula. Branch support was inferred from 100 pseudo-bootstrap replicates each. Trees were rooted at the midpoint [34] and visualized with ggtree [48]. A tree with on average higher support values is on average better resolved.

The D6 formula, which is recommended when amino-acid sequences of prokaryotic viruses are analysed, was preferentially chosen unless it presented too many contradictions with the VIRIDIC analyses and the current ICTV ranking. In this case, the D4 formula was chosen as a replacement.

Taxon boundaries at the species, genus and family level were estimated with the OPTSIL program [37], the recommended clustering thresholds [41] and an F value (fraction of links required for cluster fusion) of 0.5 [42].

This proposition of classification was confronted with dRep and VIRIDIC analyses based on nucleic acids.

dRep software [44] forms clusters with organisms that share similar DNA content based on average nucleotide identity (ANI). To do this, dRep relies on a fast primary algorithm (Mash), complemented by a more sensitive algorithm (ANIm) that is more robust to incomplete genomes. We used dRep with the default settings except for the primary clustering thresholds set to 0.7 and secondary clustering set to 0.95 to best match the thresholds set by the International Committee on Taxonomy of Viruses (ICTV) for genus and family classification [47].

According to ICTV recommendations, the phage's taxonomy was further delineated using Viral Intergenomic Distance Calculator VIRIDIC [43], (Available online: http://rhea.icbm.uni-oldenburg.de/VIRIDIC/ accessed on May 2023) which calculates the virus intergenomic distance under the BLASTn default settings. It also calculates intergenomic similarities based on the percentage of identity between two genomes determined by BLASTN. Defaults parameters were used : 95% and 70% for the species and genus threshold, respectively, and the following BLASTN parameters: '-word_size 7-reward 2-penalty-3-gapopen 5-gapextend 2'. VIRIDIC is said to be more efficient in estimating the relatedness between very distant phages that would be overestimated by other tools. Finally, this software also calculates the aligned fractions for each genome in a pair (equivalent to coverage) and the length ratio between the two genomes.

### Protein clusters analysis

We have extracted the information from the vConTACT2 PCs on which it is based to form this VC in order to analyze them in detail to select proteins which are responsible of the host *Xanthomonas* or *Xylella* specificity. Occurrences are attributed for each Protein Cluster (PC) in a Viral Cluster (VC) determinate. Core genes are PC presents in all phages of a determined VC; Soft-core genes are PCs presents in at least 95% of phages in a VC; Shell genes are the PCs presents between 5 and 95% of the considered genomes and Cloud genes are rare PCs which are present in less than 5% of the phages in a VC. Then, all the Cloud PCs are excluded from the study unless those which are presents in several VCs. All PCs with less than 50% of phages of *Xanthomonas* or *Xylella* are also excluded. PCs shared between several VCs are then studied one by one.

### RESULTS

### Phages

Phages were isolated from water samples taken from the Marseille Provence Métropole wastewater treatment plant and from rainwaters collected at the Vieux Port in Marseille, France (GPS Position 43.2941456, 5.373712).

These samples were then independently enriched with the four strains of *Xylella fastidiosa* (*Xf*) present around the mediterranean bassin: CFBP 7970 *Xylella fastidiosa fastidiosa* (*Xff*), CFBP 8402 *Xylella fastidiosa pauca (Xfp),* CFBP 8416 *Xylella fastidiosa multiplex (Xfm)* and CFBP8418 *Xfm.*

These enrichments were successively isolated three times using the overlay method on semisolid medium using *Xanthomonas albilineans* as a propagating strain.

The phage code was designed according to these steps and was constructed as follows:
- The reference to the original sample: combined sewer system (Station d'Epuration Réseau Unitaire, SERU), sanitary sewer system (Station d'Epuration Réseau Sanitaire, SERS), Vieux Port of Marseille (VP2).
- The Xf strain on which the sample was enriched: 7970, 8402, 8416, 8418.
- The (arbitrary) number of successive isolations: first isolation.second isolation.third isolation. The phages studied always result from the third isolation, which number is omitted for clarity. Finally, for ease of reference, some phages have been renamed and these names will be used hereafter (Table 4).

**Table 4: Correspondence between isolation codes and names of newly isolated phages**

| **Isolation code** | **Name** |
|---|---|
| SERS 7970 1.1 | Learch |
| SERS 8402 4.1 | Karst |
| SERS 8402 4.2 | Horst |
| SERU 7970 1.1 | Caracole |
| SERU 7970 2.1 | |
| SERU 7970 3.1 | |
| SERU 79704.1 | |
| SERU 7970 5.1 | Sov |
| SERU 7970 9a1.1 | |
| SERU 7970 9b1.1 | |
| SERU 7970 10.1 | |
| SERU 7970 11.1 | |
| SERU 7970 12.1 | |
| SERU 7970 13.1 | |
| SERU 7970 14.1 | |
| SERU 7970 15.1 | Steppe |
| SERU 8402 1.1 | Oroshi |
| SERU 8402 2.1 | |
| SERU 8402 3.1 | Darbon |
| SERU 8402 7.1 | Arval |
| SERU 8418 1.1 | Firost |
| SERU 8418 5.1 | Larco |
| SERU 8418 11.1 | Tourse |
| SERU 8418 16.1 | Boscavo |
| VP2 7970 2.1 | Aoi |
| VP2 8418 1.1 | Alme |

In this work, we also included several phages isolated previously and described in [31]. They are summarized in Table 5 and will be referred to hereafter by their name.

**Table 5: Correspondence between isolation codes and names of previously isolated phages [31]**

| **Isolation code** | **Name** |
|---|---|
| FC03 | Usme |
| FC08 | Olaya |
| FC12 | Bacata |
| FC14 | |
| FC15 | Bolivar |
| FC17 | Usaquen |
| FC23 | Cota |
| FC24 | Teja |
| FC25 | Alcala |
| FC28 | Sopo |
| FC30 | Tabio |
| FC31 | |
| FC33 | |
| FC34 | Cajica |
| FC39 | Tenjo |
| FC41 | Suba |
| FC42 | |
| FC44 | Bosa |
| FC47 | Fontebon |
| FC57 | Sumapaz |

### Clusters

### Genome de novo assembly and annotation

Assembly was performed on the following phage genomes: Learch, Karst, Horst, Caracole, SERU 7970 2.1, SERU 7970 3.1, SERU 7970 4.1, Sov, SERU 7970 9b1.1, SERU 7970 10.1, SERU 7970 11. 1, SERU 7970 12.1, SERU 7970 13.1, SERU 7970 14.1, Steppe, Oroshi, SERU 8402 2.1, Darbon, Arval, Firost, Larco, Tourse, Boscavo, Aoi, Alme.

quality assessment and cleaning of the raw reads, quality assessment of the cleaned reads, assembly of these reads followed by quality control steps, circularization of the contigs, quality assessment of the assembly, search for similarity between the phage genomes by BLAST alignment.

The sequences obtained turned out to be of good quality for all the sequenced genomes, except one (see below), and the assembly process allowed to obtain single molecule genomes compatible with the sizes of phage genomes (between 42 and 64 kbp).

Only Boscavo phage genome assembly resulted into 3 contigs (contig_1 of 47,614 bp, contig_2 of 6,984 bp, contig_3 of 6,413 bp), which could be explained by the poor quality of the sequencing reads. Given its size, contig_1 could correspond to a complete phage genome by itself, we thus checked that contigs_2 and 3 corresponded to phage sequences (presence of capsid protein encoding genes). These phage-origin contigs could be the result of a contamination by another phage. However, if this was the case, there should be a second whole genome, unless the genomes differ only in these fragments. Another round of DNA extraction and sequencing should thus be performed in the future.

Among the 25 genomes assembled, only the genomes of phages SERU 7970 12.1 and SERU 7970 14.1 were strictly identical. Phage SERU 7970 14.1 will therefore be excluded from further analysis.

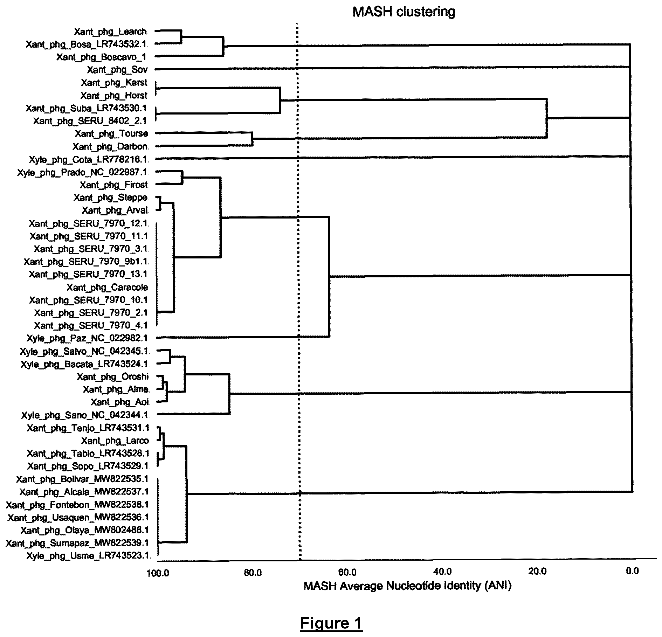

With these initial assemblies, we performed a genomic comparison tree by including the phages from our collection as well as the genomes of the four virulent phages active against *Xylella fastidiosa fastidiosa* previously characterized [28] (Figure 1) using the dRep software. dRep makes it possible to group organisms that share similar DNA content in terms of average nucleotide identity (ANI) [44]. To achieve this, dRep relies on a fast primary algorithm (Mash), complemented by a more sensitive algorithm (ANIm) that is more robust to incomplete genomes. We used dRep with the default parameters, with the exception of the primary clustering threshold set to 0.7 and the secondary clustering threshold set to 0.95 to best match the thresholds set by the International Committee on Taxonomy of Viruses (ICTV) for classification into genus and family [47].

Altogether, 42 phage genomes were included in the dataset.

According to dRep, the genomes are divided into seven completely independent branches, confirming the genetic diversity of the sequenced genomes. There are 9 groups formed by phages with more than 70% identity, suggesting 9 distinct genera according to the ICTV. The classification will be discussed in more detail in the section describing the phylogenetic description of the phages.

We then compared all these genomes with VIRIDIC [43], which is well adapted to phylogenetic classification of phages. It also calculates intergenomic similarities based on the percentage of identity between two genomes determined by BLASTN. VIRIDIC is said to be more efficient at estimating the relatedness between very distant phages that would be overestimated by other tools. We will find examples in our datasets. Finally, this software also calculates the aligned fractions for each genome in a pair (equivalent to coverage) and the length ratio between the two genomes.

### VIRIDIC classified the genomes present in the dataset into 14 genera:

1. Bosa and Learch,
2. Boscavo,
3. Sov,
4. Kart and Horst,
5. Suba and SERU 8402 2.1,
6. Tourse,
7. Darbon,
8. Cota,
9. Firost and Prado,
10. Steppe, Arval and the nine SERUs (Caracole, SERU 7970 2.1, SERU 7970 3.1, SERU 7970 4.1, SERU 7970 9b1.1, SERU 7970 10.1, SERU 7970 11.1, SERU 7970 12.1, SERU 797013.1),
11. Paz,
12. Salvo, Bacata, Aoi, Alme, Oroshi,
13. Sano,
14. Tenjo, Larco, Tabio, Sopo, Usme, Olaya, Bolivar, Usaquen, Alcala, Fontebon, Sumapaz. This confirms the over-estimation of kinship made by dRep, which formed only 9 groups with a threshold of 70%.

Based on this analysis, we decided to entrust the annotation of the Learch, Horst, Caracole, Sov, Oroshi, Darbon, Arval, Firost, Larco, Tourse, Boscavo, Aoi and Alme phages. Between 52% and 75% of the genes in each genome were functionally annotated.

Based on annotations, we were able to confirm the absence from all the sequenced genomes of problematic annotated proteins such as toxins, virulence factors and antibiotic resistance, which would hinder the use of these phages in biocontrol.

### Viral clusters and phylogenetic description of phage genomes

Below a certain level of similarity between phage genomes (65% for VIRIDIC), analyses based on proteins should be used, as they are better able to identify distant relationships [43]. In addition, comparisons based on amino acids provide a more functional viewpoint, which is important when looking for phage efficacy on a given host. We therefore used a clustering method based on distant protein homology. This method makes it possible to bring together phages that have diverged genetically but retain protein homologies and therefore have common functions.

To do this, we combined two approaches based on the whole proteome: vConTACT2 and VICTOR (whole genome analysis - amino acids). vConTACT2 compares the protein sequences of the phages studied with the BLASTp virus database. The proteins are clustered using the MCL (Markov cluster) tool. ClusterONE then clusters the viral genomes based on the MCL results. vConTACT2 was therefore used to locate our Xf and Xa infecting phages in Viral Clusters (VCs) built on the basis of shared homologous proteins.

We then used VICTOR to compare all the phages in the same VCs with each other. VICTOR performs all pairwise comparisons of the protein sequences of the phages studied using the Genome-BLAST Distance Phylogeny (GBDP) method. It then constructs a phylogenetic tree from the intergenomic distances obtained. The branch lengths of the resulting VICTOR trees are scaled in terms of these distance formulas: the D6 formula, which is recommended when amino-acid sequences of prokaryotic viruses are analyzed, was preferentially chosen unless it presented too many contradictions with the VIRIDIC analyses and the current ICTV ranking. In this case, the D4 formula was chosen as a replacement.

Taxon boundaries at the species, genus and family level were estimated with the OPTSIL program [37] with the recommended clustering thresholds [41].

This method enabled us to distribute our phages in six distinct VCs. Four of them were Siphovirus clusters VC_1, VC_2_1, VC_3, and VC_24, and two are Podovirus clusters VC_58 and VC_135.

It is interesting to put this coverage into perspective by considering the 16 VCs containing *Xanthomonas* and/or *Xylella* phages in vConTACT2. Our dataset covers 5 of the 6 VCs covering most of Xanthomonadacaes phage genomes. Each of these VC contains between 10 and 25 Xanthomonadaceae phages. The other 9 are more anecdotal with only 1 to 3 *Xanthomonas* and *Xylella* phages. The genetic diversity of the phages making up our dataset therefore appears to be fairly representative of the genetic diversity of the *Xanthomonas* and *Xylella* phages *(Xa*/*Xf* phages) characterized so far.

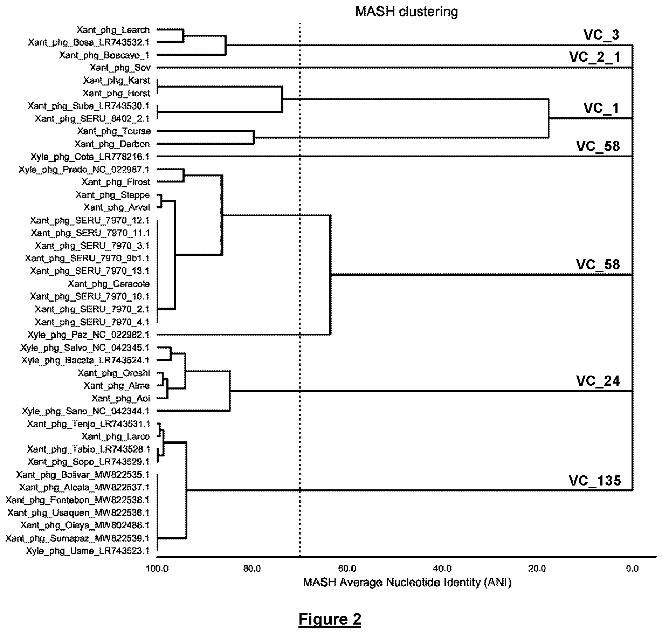

The phylogenetic tree based on DNA comparison (Figure 1) is consistent with the viral clusters formed from vConTACT2 amino acid comparisons (Figure 2).

For VC_24, we are providing a brief presentation of the VC predicted by vConTACT2, carry out a VICTOR analysis of the phages in this VC and observe in parallel the ICTV taxonomic classification of the phages already characterised.

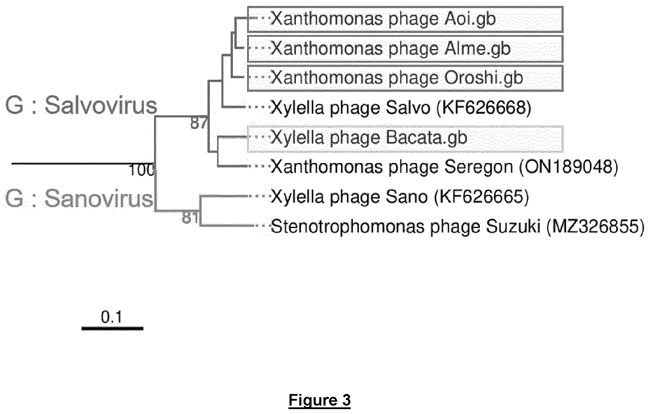

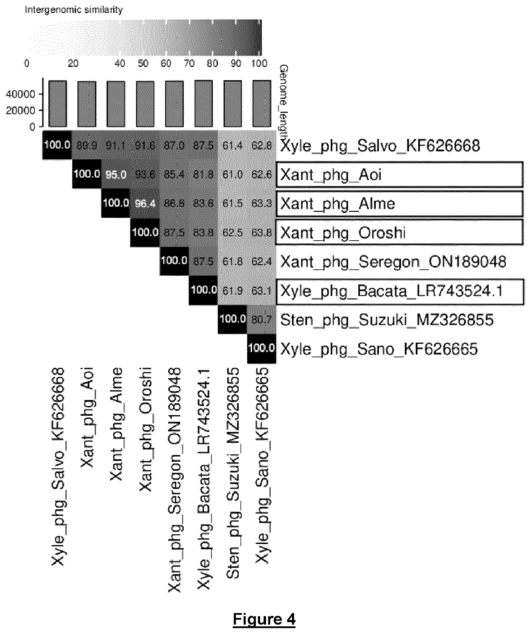

### VC_24 (Figures 3 and 4)

The small VC_24 contains only eight Siphoviruses, four of which belong to our collection: Oroshi, Aoi, Alme and Bacata. Notably, Oroshi, Aoi, Alme phage genomes are less than 92% identical to previously published Bacata genome, therefore representing a new group of phages. This VC has the particularity of containing only Xanthomonadaceae phages, 87% of which infect *Xanthomonas* and *Xylella.* More specifically, it contains one *Stenotrophomonas* phage, three *Xylella* phages (Bacata, Sano and Salvo) and four *Xanthomonas* phages.

The resulting GBDP tree has an average support of 50%. The OPTSIL grouping gave four groups of species, one genus, one family and one subfamily. The phages in this VC all belong to the family Casjensviridae, which itself contains two genera, Salvovirus and Sanovirus. Oroshi, Aoi and Alme can join Bacata within the Salvoviruses (classification confirmed by VIRIDIC).

### Viral clusters conclusions

In summary, phages from our collection are divided into six viral clusters including VC24 which comprises 8 bacteriophages.

### Protein clusters and the search for genetic determinants of efficacy against Xf

A protein cluster (PC) is a set of orthologous proteins. The aim of the work on protein clusters was to find proteins grouped within the same protein cluster that were specific to *Xanthomonas* and *Xylella* phages (Xa/Xf phages). The function of these proteins was then studied in order to determine whether they were potentially responsible for the specific efficacy of a phage against Xanthomonas and Xylella. In theory, if a protein is needed to lyse these bacteria, then it will be found in all the phages of this bacterium. This is why we are looking for protein clusters containing mainly *Xanthomonas* and *Xylella* phage proteins, which will then be considered specific, but also clusters with a good diversity of both *Xanthomonas* and *Xylella* phages.

We began by determining the occurrences of each PC in a given VC: Core genes = genes absolutely conserved in the genomes considered, Soft-core genes = genes present in at least 95% of the genomes, Shell genes = genes frequently encountered in the genomes considered, Cloud genes = very infrequent genes less than 5% of the genomes considered (Table 6).

**Table 6: Proportion of proteins shared by all phages of a given VCs (core), shared by at least 95 % of phages of a given VC (Soft-core), shared by 5-95% of phages of a given VC (Shell), or shared by less than 5% of phages of a given VC (Cloud)**

| | **Core** | **Soft Core** | **Shell** | **Cloud** |
|---|---|---|---|---|
| VC_1 (202 PCs) | 6.44 % | 4.95 % | 63.86 % | 24.75 % |
| VC_2 (151 PCs) | 9.3 % | 0 % | 90.7 % | 0 % |
| VC_3 (112 PCs) | 41.96 % | 0 % | 47.32 % | 10.71 % |
| VC_24 (104 PCs) | 50.96 % | 0 % | 49.04 % | 0 % |
| VC_58 (346 PCs) | 1.73 % | 0.58 % | 33.81 % | 63.87 % |
| VC_135 (71 PCs) | 47.89 % | 0 % | 52.11 % | 0 % |

906 PCs were derived from all the phages belonging to the 6 VCs. Intuitively, the more phages a VC contains, the more likely it is to have a high level of genetic diversity. This is why it is less likely to find a PC in all the phages of a large VC than in a small one. The % of the core genome therefore decreases in favour of the Cloud genome, which is made up of proteins that are rare in the VC under consideration, contributed by just a few individuals. If we compare the small VC_24, all the phages have almost 51% of proteins in common and 49% of shell proteins. As this VC is really small (n=8), it is not possible to have proteins in the cloud, as 1 phage alone already represents more than 12% of the phages in the VC. This highlights the difficulty of finding a suitable threshold for all the VCs. In contrast, our large VC_58 (n=38) contains only 1.7% of its PCs that are common to all phages and 64% of PCs that are part of the Cloud genome, highlighting that the greater number of individuals in this VC is also accompanied by greater genetic diversity.

There are PCs shared between several VCs (Table 7) but there is no common PC between all the *Xa*/*Xf* phages or between the six VCs concerned in this study. There is therefore no protein specific to phages infecting Xanthomonas and/or *Xylella* that would be absent from other phages. To process all the data, the PCs were first sorted. All the PCs from the cloud genomes of the VCs were discarded except when they were present in several VCs.

First, we looked at the PCs shared by several VCs in our study. A total of 91 PCs are shared: 58 are present in 2 of our VCs, 12 in 3 of our VCs and 3 in 4 of our VCs. It should be noted that there are no PCs present in 5 or 6 different VCs in our study. Based on the hypothesis that a protein important for host specificity should be found in as many *Xylella* / *Xanthomonas* phages as possible and in few phages infecting other bacterial species, we sorted the PCs again. PCs containing less than 50% *Xanthomonas* or *Xylella* phages were discarded. After this sorting, 17 PCs remained present in two of our VCs, two present in three of our VCs and two present in four of our VCs.

PCs shared by 4 VCs in our study:
PCs PC001115 and PC001081 are promising. They are shared between VC_1, VC_2_1, VC_58 and VC_135 but also include phages from VC_10 consisting of 2 phages (Stenotrophomonas_phage_Pokken and Xanthomonas_phage_RiverRider) and other phages not associated with a VC (2 Pseudomonas phages, 1 Xanthomonas phage common to both PCs and 1 Colwellia phage for PC001081 only). These two PCs are annotated "tail fiber". Tail fibers enable recognition and adsorption of the phage to its cellular host and are therefore crucial for host specificity [50]. These two PCs possess 85% and 81% of Xanthomonas and Xylella phages (Table 7). It is important to note that among the phages of these PCs that are not Xa/Xf phages, there are 4 and 5 phages of Stenotrophomonas, which is a bacterium that also belongs to the Xanthomonadaceae family. What's more, we can see that not all the phages in a VC, which are therefore close to each other, are necessarily included in a PC. It is interesting to see which phages of the VCs concerned by a PC are not included in that PC. The sum of the phages in the VCs covered by PCs 001115 and 001081 is 87 and 88. These PCs include 41 and 42 phages respectively, so they exclude 46. 83% of them and 80% of these 46 excluded phages are phages infecting other bacteria. This PC therefore discriminates between *Xa*/*Xf* phages and others among phages that are very close genetically. This shows that these PCs are not necessary (because some *Xanthomonas* and *Xylella* phages are excluded) but are still very specific.

We wanted to find out whether it was possible to differentiate the proteins of Xa/Xf phages from the proteins of phages with other hosts within a PC. We carried out an alignment of the amino acid protein sequences of each phage. Xa/Xf phages are not clearly separated from phages with other hosts (data not shown). One may wonder about the host range of the phages concerned: are these *Stenotrophomonas* or *Pseudomonas* phages capable of infecting certain strains of *Xanthomonas* and/or *Xylella?* This is the case of two virulent phages belonging to the Autographiviridae family capable of infecting pathovars of the *Pseudomonas* and *Xanthomonas* genera [49]. In this case, it would be impossible (and probably irrelevant) to differentiate these proteins.

PCs shared by 3 VCs in the study:
Of the 12 PCs common to three of our VCs, only two have more than 50% Xa/Xf phages and are annotated 'tail assembly protein'. A priori, this annotation does not seem to explain either host specificity or efficacy against *Xylella.* PC002866 includes 19 phages from VCs 1, 3 and 24, 12 (63%) of which are Xa/Xf phages. However, it excludes 14 (56%) *Xanthomonas* or *Xylella* phages present in the VCs covered by this PC. Similar results were found for PC002580, which has 57% Xa/Xf phages (12) but excludes 54% of the Xa/Xf phages (14) present in the VCs covered by this PC. These two PCs are therefore of limited interest for our research.

PCs shared by 2 VCs in the study:
Of the 17 PCs that include phages spread across 2 VCs in our study and have more than 50% Xanthomonas or Xylella phage, we can already rule out one that includes only 2 phages and is too small to be of interest.

On the other hand, one PC is very interesting in terms of its size and percentages: PC001312. It has 31/35 (88%) Xa/Xf phages distributed in VCs 58, 135 and 10. The 19 phages in these VCs not included in this PC are all phages from another bacterium. This PC also has a tail fibre annotation.

These two cases, which are relatively easy to interpret, are exceptions. Analysis of the other PCs was more difficult. For example, PC010399 seems interesting at first sight: it has 100% Xa/Xf phage and excludes only 41%, but this actually corresponds to 4 Xa/Xf phages included and 24 excluded. It can therefore be considered of little interest. To compensate for this bias in percentages, we could focus on the amount of phage: number of *Xa*/*Xf* phage included - excluded. Using this method, the second best PC after 001312 described above is 002309. However, when looking more closely, numbers are less convincing: with 62% of *Xa*/*Xf* phage (15/24) included in this PC and 57% (4/7) of *Xa*/*Xf* phage excluded, which suggests a poor specificity.

This highlights the complexity of the analysis and the importance of putting all the data into perspective for the analysis of other PCs which are smaller, have less marked percentages or exclude a significant number *of Xanthomonas* or *Xylella* phages.

**Table 7: Phage composition of PCs shared by several VCs. Phages from these VCs that are excluded from the PCs. VCs in bold correspond to VCs including phages from our collection.**

| | | **Phages represented in the PC** | | | | | **Phages excluded from PC** | | |
|---|---|---|---|---|---|---|---|---|---|
| **PC PC annotation** | | **nb phage s in PC** | **% *Xa*/*Xf* phage s** | **% phg other specie s** | **VCs with at least one protei n in this PC** | **nb of phage s in all VCs** | **nb of phages exclude d** | **% phag e *Xa*/*Xf*** | **% phage other specie s** |
| | | | | | | | | | |
| **PC0011 15** | Tail fiber | 41 | 85.4 | 14.6 | **1, 2_1, 58, 135, 10** | 87 | 46 | 17.4 | 82.6 |
| **PC0010 81** | Tail fiber | 42 | 81.0 | 19.0 | **1, 2_1, 58, 135,** 10 | 88 | 46 | 19.6 | 80.4 |
| **PC0028 66** | tail assembly | 19 | 63.2 | 36.8 | **1, 3, 24** | 44 | 25 | 56.0 | 44.0 |
| **PC0025 80** | tail assembly | 21 | 57.1 | 42.9 | **1, 3, 24,** 9 | 47 | 26 | 53.8 | 46.2 |
| **PC0261 41** | Hypothetical | 2 | 50.0 | | | | | | |
| **PC0127 53** | Tail | 5 | 80.0 | 20.0 | **1, 2_1** | 30 | 25 | 28.0 | 72.0 |
| **PC0104 72** | Hypothetical | 6 | 83.3 | 16.7 | **3, 135** | 27 | 21 | 85.7 | 14.3 |
| **PC0104 09** | tail fiber | 6 | 83.3 | 16.7 | **1, 2_1** | 30 | 24 | 25.0 | 75.0 |
| **PC0104 08** | putative tail | 6 | 83.3 | 16.7 | **1, 2_1** | 30 | 24 | 25.0 | 75.0 |
| **PC0103 99** | L-shapped tail fiber | 4 | 100.0 | 0.0 | **1, 58,** 10 | 63 | 59 | 40.7 | 59.3 |
| **PC0090 88** | Hypothetical | 7 | 85.7 | 14.3 | 1, 2_1 | 30 | 23 | 21.7 | 78.3 |
| **PC0090 86** | Hypothetical | 7 | 57.1 | 42.9 | **1, 58** | 61 | 54 | 40.7 | 59.3 |
| **PC0080 37** | Tail / putative cell wall peptidase | 8 | 75.0 | 25.0 | **1, 2_1** | 31 | 23 | 21.7 | 78.3 |
| **PC0055 67** | Hypothetical | 10 | 90.0 | 10.0 | **1, 24,** 138 | 33 | 23 | 39.1 | 60.9 |
| **PC0051 09** | Hypothetical | 12 | 100.0 | 0,0 | **24, 135** | 22 | 10 | 90.0 | 10.0 |
| **PC0047 18** | Hypothetical | 14 | 64.3 | 35.7 | **1, 3** | 36 | 22 | 45.5 | 54.5 |
| **PC0033 97** | Hypothetical / RNA pol bindind | 18 | 55,6 | 44.4 | **1, 2_1** | 30 | 12 | 8.3 | 91.7 |
| **PC0033 96** | Hypothetical | 14 | 100.0 | 0.0 | **1, 58** | 61 | 47 | 25.5 | 74.5 |
| **PC0027 11** | tail fiber | 21 | 66.7 | 33.3 | **3, 24,** 138, 246 | 29 | 8 | 50.0 | 50.0 |
| **PC0023 09** | tail assembly | 25 | 62.5 | 37.5 | **3, 24,** 138 | 31 | 7 | 57.1 | 42.9 |
| **PC0013 12** | Tail fiber | 35 | 88.6 | 11.4 | **58, 135,** 10 | 54 | 19 | 0.0 | 100.0 |

### Protein clusters conclusions

In summary, the most convincing PCs were VC_1, VC_2_1, VC_58 and VC_135 for PCs 001115 and 001081 and VCs 58 and 135 for PC 001312. As these PCs are tail fiber clusters, we thus looked for interesting PCs with this annotation for our last two VCs 3 and 24.

There are 2 additional PCs with a Tail Fiber annotation for VC_3. The first is very limited (3 phages) and does not contain any phage from our collection and is therefore of no immediate interest. The second (PC009070) contains 7 phages from VC_3 alone, including 5/7 *Xa*/*Xf* phages (71%). However, of the phages from this VC that do not belong to this PC, 4/6 are *Xanthomonas* phages.

All the phages in VC_24 include a protein clustered in PC005121 annotated Tail Fiber containing 11 phages. 9/11 phages in this PC are *Xa*/*Xf* phages (82%), the other two are *Stenotrophomonas* phages. This PC is therefore also of interest.

### Microscopy

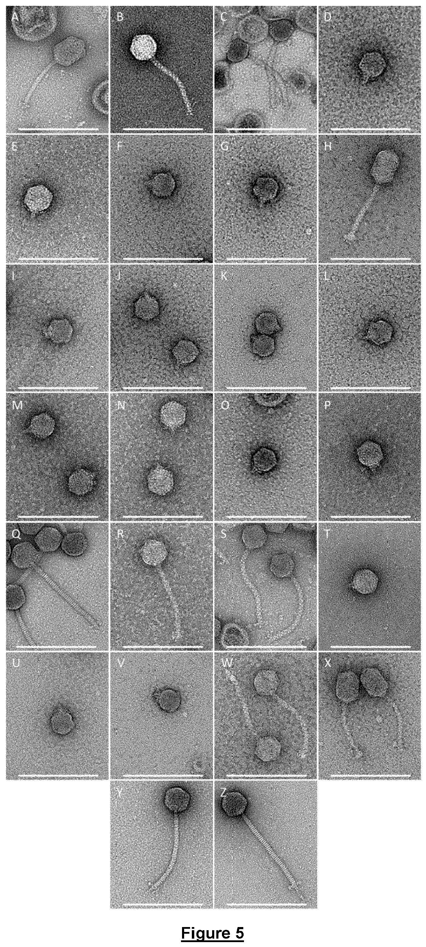

The morphological characterisation of our isolated phages was carried out using patches isolated on a double layer of agar, negative staining and observed by Transmission Electron Microscopy (TEM). Our phages are no exception to the *Xanthomonas* phages already characterised to date and belong to the phages with tails with podovirus morphologies - small non-contractile tail - (Caracole, SERU 7970 2.1, SERU 7970 3.1, SERU 7970 4.1, SERU 7970 9a1. 1, SERU 7970 9b1.1, SERU 7970 10.1, SERU 7970 11.1, SERU 7970 12.1, SERU 7970 13.1, SERU 7970 14.1, Steppe, Arval, Firost, Larco) and siphoviruses - long non-contractile tail - (Learch, Karst, Horst, Sov, Oroshi, SERU 8402 2.1, Darbon, Tourse, Boscavo, Aoi, Alme) (Figure 5).

All have a classic icosahedral capsid morphology, except for Boscavo, Learch and Sov, which have an elongated capsid. While there is no doubt about the striated nature of the phage tails for phages Q. Oroshi, Y Aoi and Z. Alme, whose tails end in a point surmounted by small fibers, it is more difficult to determine for the other siphoviruses and certainly depends on the quality of the preparations. However, based on all the observations made on each of our phages, we can conclude that striations are present on all the tails of newly isolated phage siphoviruses, whatever the capsid morphology.

To further characterize the morphology of our phages, we measured capsid size and tail length using imaged software. The measurements are reported in Table 8. For this analysis, we included the other *Xanthomonas* and *Xylella* phages present in our collection and characterized in the publication [31] (Usme, Olaya, Bacata, FC14, Bolivar, Usaquen, Teja, Alcala, Tabio, FC31, FC33, Caljica, Tenjo, Suba, FC42, Bosa, Fontebon, Sumapaz). These phages have an icosahedral capsid podovirus morphotype (Usme, Olaya, Bolivar, Usaquen, Teja, Alcala, Tabio, FC33, Caljica, Tenjo, Fontebon, Sumapaz), icosahedral capsid siphovirus (Bacata, FC14, FC31, Suba, FC42) and elongated capsid siphovirus (Bosa).

**Table 8: Morphological characteristics of the phages in our collection**

| **Phage** | **Morphotype** | | **Capsid shape** | **Average Capsid length nm** | **Average Tail length nm** |
|---|---|---|---|---|---|
| Learch - SERS 7970 1.1 | Siphovirus | B2 | Elongate | 89.4 ± 3.3 (n=87) | 145.4 ± 7.6 (n=60) |
| Karst - SERS 8402 4.1 | Siphovirus | B1 | Icosahedral | 64.8 ± 2.7 (n=29) | 177.4 ± 6.2 (n=21) |
| Karst - SERS 8402 4.1 | Siphovirus | B1 | Icosahedral | 67.2 ± 2.2 (n=15) | 177.0 ± 4.7 (n=15) |
| Horst - SERS 8402 4.2 | Siphovirus | B1 | Icosahedral | 59.2 ± 3.1 (n=23) | 180.4 ± 5.6 (n=16) |
| Caracole - SERU 7970 1.1 | Podovirus | C1 | Icosahedral | 63.4 ± 3.4 (n=12) | 14.4 ± 4.6 (n=9) |
| SERU 7970 2.1 | Podovirus | C1 | Icosahedral | 66.3 ± 2.8 (n=34) | 16.9 ± 4.0 (n=27) |
| SERU 7970 3.1 | Podovirus | C1 | Icosahedral | 60.6 ± 3.7 (n=24) | 17.8 ± 3.8 (n=16) |
| SERU 7970 4.1 | Podovirus | C1 | Icosahedral | 61.3 ± 4.5 (n=14) | 18.1 ± 3.4 (n=11) |
| Sov - SERU 7970 5.1 | Siphovirus | B2 | Elongate | 89.2 ± 2.5 (n=23) | 160.2 ± 6.3 (n=23) |
| SERU 7970 9a1.1 | Podovirus | C1 | Icosahedral | 63.0 ± 2.7 (n=7) | 14.2 ± 1.8 (n=6) |
| SERU 7970 9b1.1 | Podovirus | C1 | Icosahedral | 61.5 ±3.0 (n=50) | 17.5 ± 3.3 (n=22) |
| SERU 7970 10.1 | Podovirus | C1 | Icosahedral | 57.7 ±3.2 (n=70) | 19.2 ± 2.7 (n=38) |
| SERU 7970 11.1 | Podovirus | C1 | Icosahedral | 62.0 ± 3.3 (n=37) | 21.5 ± 2.7 (n=20) |
| SERU 7970 12.1 | Podovirus | C1 | Icosahedral | 61.7 ± 2.8 (n=34) | 21.9 ± 2.2 (n=21) |
| SERU 7970 13.1 | Podovirus | C1 | Icosahedral | 67.6 ± 2.6 (n=63) | 23.3 ± 3.3 (n=17) |
| SERU 7970 14.1 | Podovirus | C1 | Icosahedral | 58.9 ± 2.9 (n=35) | 22.0 ± 2.8 (n=12) |
| Steppe - SERU 7970 15.1 | Podovirus | C1 | Icosahedral | 63.2 ± 2.9 (n=17) | 20.3 ± 3.1 (n=9) |
| Oroshi - SERU 8402 1.1 | Siphovirus | B1 | Icosahedral | 65 ± 3.2 (n=118) | 239.1 ± 7.6 (n=91) |
| SERU 8402 2.1 | Siphovirus | B1 | Icosahedral | 66.7 ± 2.6 (n=50) | 197.9 ± 6.5 (n=51) |
| Darbon - SERU 8402 3.1 | Siphovirus | B1 | Icosahedral | 62.7 ± 3.6 (n=136) | 210.4 ± 6.6 (n=101) |
| Arval - SERU 8402 7.1 | Podovirus | C1 | Icosahedral | 64.8 ± 2.1 (n=46) | 20.4 ± 3.0 (n=30) |
| Firost - SERU 8418 1.1 | Podovirus | C1 | Icosahedral | 61.1 ± 3.8 (n=41) | 20.7 ± 2.2 (n=12) |
| Larco - SERU 8418 5.1 | Podovirus | C1 | Icosahedral | 57.4 ± 2.5 (n=37) | 18.8 ± 3.4 (n=10) |
| Tourse - SERU 8418 11.1 | Siphovirus | B1 | Icosahedral | 65.2 ± 3.0 (n=74) | 196.4 ± 7.3 (n=52) |
| Boscavo - SERU 8418 16.1 | Siphovirus | B2 | Elongate | 85.0 ± 2.9 (n=88) | 146.6 ± 5.3 (n=74) |
| Aoi - VP2 7970 2.1 | Siphovirus | B1 | Icosahedral | 64.7 ± 3.2 (n=33) | 238.1 ± 7.6 (n=26) |
| Alme - VP2 8418 1.1 | Siphovirus | B1 | Icosahedral | 65.4 ± 2.9 (n=54) | 236.4 ± 8.1 (n=39) |
| Usme - FC03 | Podovirus | C1 | Icosahedral | 65.0 ± 2.4 (n=16) | 12.5 ± 1.7 (n=10) |
| Olaya - FC08 | Podovirus | C1 | Icosahedral | 65.8 ± 1.3 (n=9) | 9.5 ± 2.3 (n=8) |
| Bacata - FC12 | Siphovirus | B1 | Icosahedral | 70.2 ± 2.6 (n=15) | 226.7 ± 4.0 (n=15) |
| FC14 | Siphovirus | B1 | Icosahedral | 70.7 ± 3.0 (n=9) | 226.5 ± 3.6 (n=10) |
| Bolivar - FC15 | Podovirus | C1 | Icosahedral | 66.8 ± 1.3 (n=6) | 13.5 ± 1.3 (n=5) |
| Usaquen - FC17 | Podovirus | C1 | Icosahedral | 61.0 ± 2.6 (n=13) | 17.8 ± 3.4 (n=13) |
| Teja - FC24 | Podovirus | C1 | Icosahedral | 68.3 ± 2.8 (n=45) | 14.5 ± 3.0 (n=34) |
| Alcala - FC25 | Podovirus | C1 | Icosahedral | 67.2 ± 4.2 (n=6) | 18.0 ± 2.7 (n=6) |
| Tabio - FC30 | Podovirus | C1 | Icosahedral | 67.9 ± 3.2 (n=6) | 13.5 ± 2.1 (n=3) |
| FC31 | Siphovirus | B1 | Icosahedral | 69.5 ± 5.4 (n=16) | 190.4 ± 4.5 (n=19) |
| FC33 | Podovirus | C1 | Icosahedral | 68.9 ± 2.6 (n=43) | 14.2 ± 3.3 (n=19) |
| Cajica - FC34 | Podovirus | C1 | Icosahedral | 68.0 ± 3.3 (n=37) | 16.5 ± 3.8 (n=24) |
| Tenjo - FC39 | Podovirus | C1 | Icosahedral | 64.0 ± 1.5 (n=6) | 24.0 ± 4.4 (n=6) |
| Suba - FC41 | Siphovirus | B1 | Icosahedral | 64.9 ± 3.0 (n=31) | 202.7 ± 4.3 (n=32) |
| FC42 | Siphovirus | B1 | Icosahedral | 65.1 ± 3.4 (n=27) | 194.0 ± 4.3 (n=13) |
| Bosa - FC44 | Siphovirus | B2 | Elongate | 91.2 ± 1.2 (n=12) | 148.8 ± 3.6 (n=11) |
| Fontebon - FC47 | Podovirus | C1 | Icosahedral | 62.9 ± 2.7 (n=17) | 16.8 ± 3.3 (n=15) |
| Sumapaz - FC57 | Podovirus | C1 | Icosahedral | 62.2 ± 1.9 (n=11) | 15.7 ± 3.0 (n=11) |

Whatever the podovirus or siphovirus morphotype, the size of icosahedral capsids ranged from 57.4 ± 2.5 nm (Larco) to 70.7 ± 3 nm (FC14). The elongated capsids have an average length of 85.0 ± 2.9, 89.2 ± 2.5, 89.4 ± 3.3, 91.2 ± 1.2 nm for the Boscavo, Sov, Learch and Bosa phages respectively. This raises the question of a link between capsid size and genome size. Icosahedral capsid size is not correlated with genome size. However, phages with elongated capsids have the largest genomes. The quality of the sequencing of the Boscavo phage only allowed an assembly fragmented into 3 contigs of 47,614 bp, 6,984 bp and 6,413 bp. While the two small contigs are indeed of phage origin, it is difficult to determine whether they actually belong to Boscavo. The size of the Boscavo genome is therefore between 47614 and 61011 bp. Sov has a genome of 62014 bp, Learch 63771 bp and Bosa 63828 bp.

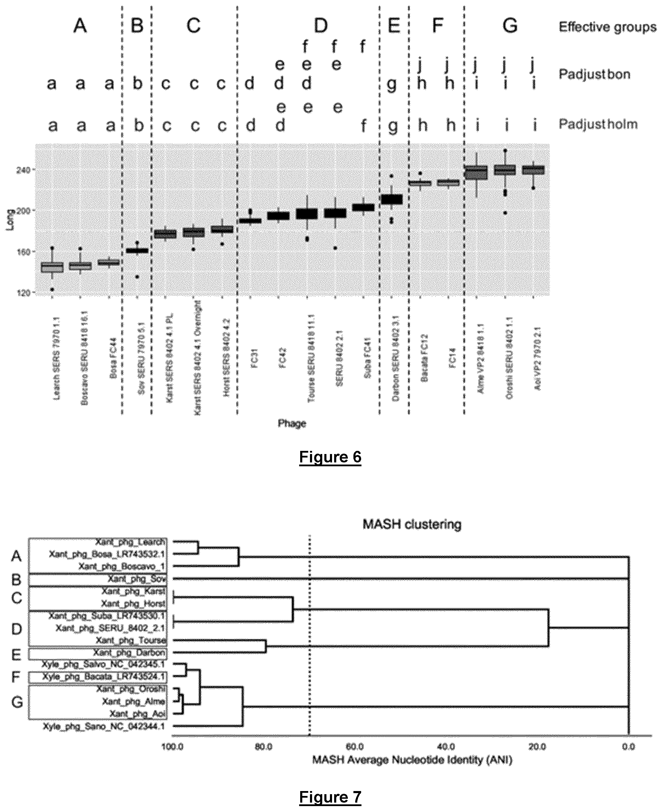

As the tail lengths of podoviruses cannot be measured properly, the measurements are given for information only. However, all siphovirus tail lengths were measured and compared using statistical tools. As the quantitative variable (tail length) did not follow a normal distribution for all the populations compared (shapiro-Wilk test p-value « 0.1, therefore rejecting hypothesis H0: the sample follows a normal distribution), we performed the non-parametric Kruskall-Wallis test followed by the non-parametric Wilcoxon post hoc test (Figure 6). The Bonferroni and Holm corrections were used to adjust the p-value.

The Kruskall-Wallis test indicates that not all samples are equivalent (p-value < 2.2e-16). Depending on the p-value adjustment chosen for the Wilcoxon test, the statistical groups differ slightly. We can identify seven distinct groups: Group A consists of Learch, Boscavo and Bosa; Group B is represented solely by Sov; Group C corresponds to the Karst and Horst phages; group D includes SERU 8402 2.1, Tourse, FC31, Suba and FC42, and group E is represented solely by Darbon; Group F includes Bacata with FC14; Group G includes Oroshi, Aoi and Alme.

Groups A, B, C and E are clearly defined whatever the p-value adjustment. Groups F and G, clearly distinct in the case of the Holm adjustment, are more ambiguous in the case of the Bonferroni adjustment: while Bacata and FC14 are different from Oroshi and Aoi and vice versa, Alme is not statistically different from any of the other 4 (p value = 1.000 Aoi, 0.057 Bacata, 0.319 FC14, 1.000 Oroshi) with this adjustment. We decide to separate groups F and G which are effectively separated with the Holm adjustment as well as for most of the good fit comparisons.

Finally, group D is ambiguous whatever the p-value adjustment. With Holm adjustment, it is separated into three subgroups: FC31 and FC42, FC 42 with Tourse and SERU 8402 2.1 and a final Suba group. The Bonferroni adjustment also distinguishes 3 subgroups: FC31, FC42, Tourse for the first, FC42, Tourse, SERU 8402 2.1 for the second and Tourse, SERU 8402 2.1 and FC41 for the third. As these subgroups differ according to the statistical method, with phages in several subgroups, we have grouped them together in a well-defined group D for greater clarity.

If we put this analysis into perspective with the VCs previously obtained, we can see that each siphovirus VC is represented by one or more adjacent groups with statistically different tail lengths. Group A corresponds to VC_3, group B corresponds to VC_2_1, groups C, D and E belong to VC_1 and groups F and G belong to VC_24.

To take the analysis a step further, we superimposed the tail length statistical groups on the phage genome comparison tree (Figure 7). Sov, alone in its statistical group, is branched alone in the tree. Learch, Boscavo and Bosa are also well branched together and grouped in their tail length statistical group. The analysis of tail lengths makes it possible to distinguish two distinct branches belonging to the same main branch corresponding to VC_24. On one side we find Bacata and FC14 (FC14 is genetically 100% identical to Bacata and is therefore not represented on the tree) and on the other Oroshi, Aoi and Alme. In VC_1, the tail length groups separate Horst and Karst (group C) from the rest of the VC and also Darbon (group E) from the rest of the VC.

Analysis of tail lengths does not give any further indication of the rest of VC_1 between phages FC31, FC42, Tourse, SERU 8402 2.1 and Suba (FC42 is genetically very close to Suba and FC31 has not been sequenced).

Note that the groups of tail lengths do not allow us to assume a % difference between the phage genomes. For example, Oroshi and Bacata stand out even though they are 91.706% identical out of 87.306% coverage, but Tourse and Suba, which are further apart (17.38% identical), are grouped together in the same tail-length statistical group. It is also interesting to note that the tail length measurements are extremely close on independent samples from identical phages: Karst was measured twice as part of a sample preparation protocol optimisation and its measurements are 177.4 ± 6.2 (n=21) and 177.0 ± 4.7 (n=15) and Bacata and FC14 are 100% genetically identical and their measurements are 226.7 ± 4.0 (n=15) and 226.5 ± 3.6 (n=10) respectively.

### Efficiency tests Results

### Overlays

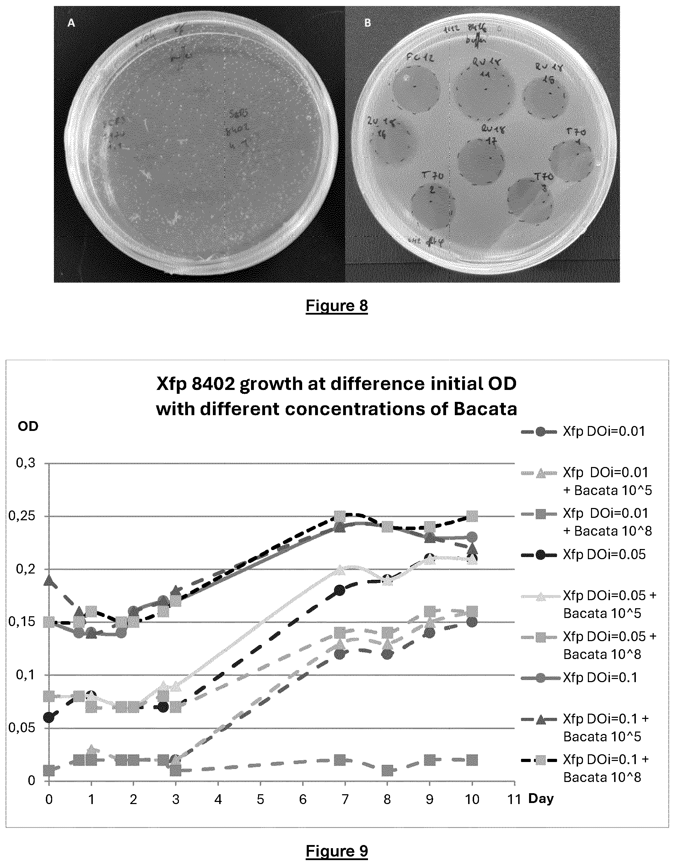

As *Xylella* has a strong tendency to aggregate, the overlay allows the bacteria to grow more homogeneously and avoids the formation of granules, which makes the effectiveness of the phages less visible (Fig. 8). Optimization was crucial to obtain homogenous overlays and, to our knowledge, it was the first time that environmental *Xylella* strains were grown in overlays.

For each strain, at least 1 phage of each VC has been tested in triplicate. A test was considered positive when inhibition of the bacterial overlay could be observed at the phage spot, otherwise it was considered negative. These results are therefore not quantitative. To summarize all the tests carried out, an efficacy frequency was determined for each tested phage (positive test/number of tests). Three levels were determined from these frequencies:
- Non-efficient [0, 0.4[
- Mitigate [0.4, 0.5]
- Efficient ]0.5, 1]

**Table 9: Summary of efficacy frequencies of each phage VC_24 in overlays against the 2 Xylella subspecies. E = efficient (frequency between 50 and 100%), M = mitigate (frequency between 40 and 50%)**

| VC | Phage | Efficacy frequencies overlay 7970 | | Efficacy frequencies overlay 8416 | |
|---|---|---|---|---|---|
| VC_24 | Bacata | 0.4 | M | 0.7 | E |
| VC_24 | Alme | 0.5 | M | 0.8 | E |
| VC_24 | Oroshi | 0.5 | M | 0.9 | E |
| VC_24 | Aoi | 0.5 | M | 0.8 | E |
| | Xylphi- PD^{™} | 0.6 | E | 1.0 | E |

Strain *Xfm* 8416 is more sensitive than Xff 7970. Against *Xff,* VC_24 shows efficacy: phages showed a frequency between 40 and 50% efficiency over 14, 4, 8, 4 tests for Bacata, Alme, Oroshi and Aoi, respectively. In comparison, Xylphi- PD^{™} was effective in only 3 out of 5 cases.

### Kinetics in liquid media

### Proof of concept

Despite various attempts to optimize *Xylella* growth in liquid media, optimal parameters could not be identified. Agitation at 160 rpm and initial OD₆₀₀ do not seem to be decisive parameters for growth, as they make no significant difference once growth has been observed. The key to ensuring *Xf* growth in liquid media could lie in optimizing the preculture of *Xylella* on plates: number of replicates, number of days after the last replicate, etc.

A total of 7 infection experiments with different phages were carried out. *Xff* grew 6 times, 5 times for 8402, 4 times for 8416 and 6 times for 8418.

In an experiment comparing different initial OD₆₀₀ and phage concentrations (Bacata - FC12), we concluded that the lower the initial OD₆₀₀ and the higher the phage concentration, the greater the growth inhibition (Figure 9).

With the highest initial OD₆₀₀, the phage did not inhibit growth in the overlay regardless of the concentration added. However, with an initial OD₆₀₀ of 0.05 of *Xfp,* Bacata at 10⁸ PFU/mL allows a decrease from 0.21 to 0.16, i.e. a decrease of 0.05 OD₆₀₀ units at 10 days. With an initial OD₆₀₀ of 0.01, this phage concentration totally inhibits bacterial growth.

Another point revealed by our tests is that the absence of a decrease in OD₆₀₀ does not exclude a relative efficacy of the phage. In fact, checking phage production in the presence of the bacteria confirms that the phage recognizes and replicates. We carried out this measurement on three of the seven tests. The phage's ability to increase its titre depends on the initial quantity added. In fact, given the previous observation, phages are added in large quantities and are already at a plateau. This plateau seems to differ according to the Xf strain tested. The initial phage concentration sufficient to see a reduction in OD₆₀₀ but low enough to be able to quantify an increase in titre has yet to be defined. Finally, among the phages titrated, those that showed an increase in titer were Bacata, Arval and Xylphi-PDTM. These observations need to be confirmed. To gain a better understanding of the dynamics between OD₆₀₀, bacterial numbers and phages, we have begun developing digital PCR on *Xf,* after having developed it on Xa. Note that Xylphi-PD^{™} only prevent growth of 8416, with the lowest concentration out of the two tested, suggesting that the optimum concentration is not always the highest.

### Liquid tests

Different times after Xf infection were tested for phage addition. In these tests, a phage is tested against all four Xf with addition at day 0, 4 and 7 after Xf inoculation. We chose to add the phages at the same time as the bacteria, as this seemed more favorable, and the manipulation was redesigned to test a set of phages against one of the Xf strains, making it possible to compare them. In these experiments, streptomycin was added as a positive control at a concentration of 20 mg/mL.

Phages tested were: Cota, Horst, Karst, Darbon, Learch, Bacata, Alme, Oroshi, Aoi, Arval, Usme and Xylphi-PD^{™}.

### Review

To summarize all these tests and results, we have determined a threshold value above which a phage effect was observed. This threshold consists of a difference in OD₆₀₀ of at least 0.05 between the condition with phage and the control condition without phage, whether we observed a decrease (efficiency) or an increase. This threshold is chosen to correspond as closely as possible to the growth of the four strains, to be high enough to avoid non-significant differences, and low enough to allow differences to be detected. As an indication, with this threshold 28/66 tests exceed this threshold (42%) for 7970, 20/31 (65%) for 8402, 24/37 (65%) for 8416 and 8/23 (26%) for 8418. A second threshold of 0.1 has been chosen as a guideline, so that phages can be compared with each other in tests comparing several phages on the same strain.

Using the 0.05 threshold, we distinguished three trends to characterize the efficacy of a phage in liquid medium (Table 10):
- Non-efficient: the phage has shown no efficacy to date (no difference and/or increase in OD₆₀₀ above the first threshold).
- Mitigated: the phage has already shown a reduction but also an increase in OD₆₀₀
- Efficient: phage has already shown efficacy

**Table 10: Summary of efficacy of each phage VC24 in liquid media against the 4 Xylella subspecies. Considering our thresholds: X = showed efficiency a least one time, Y = showed efficiency but also antagonistic effect, Z = showed no efficiency and/or antagonistic effects. The numbers indicate the number of times the phage has been tested against the strain.**

| VC | Phage | Efficacy on 7970 liquid growth | | Efficacy on 8402 liquid growth | | Efficacy on 8416 liquid growth | | Efficacy on 8418 liquid growth | |
|---|---|---|---|---|---|---|---|---|---|
| VC_24 | Bacata | 8 | Y | 4 | Y | 3 | X | 4 | X |
| VC_24 | Alme | 4 | Z | 2 | X | 2 | X | 1 | Z |
| VC_24 | Oroshi | 6 | Z | 3 | X | 4 | X | 2 | X |
| VC_24 | Aoi | 6 | Y | 2 | Z | 4 | Y | 2 | Z |
| | Xylphi-PD^{™} | 6 | Z | 3 | X | 4 | X | 2 | X |

### Biofilms

As the biofilm tests mirrored the liquid media tests in 96-well plates, the same bacterial precultures and cultures were used. Similarly, the same difficulties were encountered with bacterial growth, whether in preculture or during testing.

To summarize all these results, we have determined a threshold value above which a phage effect is noted. This threshold consists of a difference in OD₆₀₀ of at least 0.2 between the condition with phage and the control condition without phage, whether a decrease (efficiency) or an increase. As before, this threshold is chosen to correspond as closely as possible to the biofilm production of the four strains, to be high enough to avoid non-significant differences, and low enough to allow differences to be detected. As an indication, 27/55 tests exceeded this threshold (49%) for 7970, 15/22 (68%) for 8402, 11/33 (33%) for 8416 and 3/12 (25%) for 8418. A second threshold of 0.4 has been chosen as a guideline, so that phages can be compared with each other in tests comparing several phages on the same strain.

Using the 0.2 threshold, we distinguished three trends to characterize the efficacy of a phage to reduce biofilm formation (Table 11):
- Non-efficient: the phage has shown no efficacy to date (no difference and/or increase in OD₆₀₀ above the first threshold).
- Mitigated: the phage has already shown a reduction but also an increase in OD₆₀₀
- Efficient: phage has already shown efficacy

**Table 11: Summary of efficacy of each phage VC24 against biofilm formation of the 4 Xylella subspecies. Considering our thresholds: X = showed efficiency a least one time, Y = showed efficiency but also antagonistic effect, Z = showed no efficiency and/or antagonistic effects. The numbers indicate the number of times the phage has been tested against the strain.**

| VC | Phage | Efficacy on 7970 biofilm | | Efficacy on 8402 biofilm | | Efficacy on 8416 biofilm | | Efficacy on 8418 biofilm | |
|---|---|---|---|---|---|---|---|---|---|
| VC_24 | Bacata | 5 | Y | 2 | X | 3 | X | 1 | X |
| VC_24 | Alme | 5 | Z | 2 | X | 3 | X | 1 | X |
| VC_24 | Oroshi | 5 | Z | 2 | Z | 3 | X | 1 | Z |
| VC_24 | Aoi | 4 | X | 1 | X | 3 | X | 1 | Z |
| | Xylphi-PD^{™} | 5 | Z | 2 | X | 3 | X | 1 | Z |

The subspecies least sensitive to phages under biofilm conditions appears to be *Xff* 7970, but in this assay *Xfp* 8402 is the most sensitive.

VC24 phages was tested a minimum of three times for 7970, twice for 8402 and 8416 and once for 8418.

On 8402, Bacata, Alme, Aoi, and Xylphi-PD^{™} reduced OD₆₀₀ by at least 0.2 but less than 0.4, and Oroshi failed to reduce OD₆₀₀ beyond the first threshold.

Out of 8416, for the first test comparing several phages, only the Oroshi phage reduced OD₆₀₀ more than 0.2 units, and for the second, only the VC_24 phages significantly reduced biofilm formation above the first threshold for Bacata and the second for Alme, Oroshi and Aoi, as well as Xylphi-PD^{™}.

It should be noted that Xylphi-PD^{™} never showed a reduction in biofilm production by either 7970 or 8418, unlike some of our phages tested alone.

### REFERENCES

1. EFSA Panel on Plant Health (EFSA PLH Panel) Jeger M., Caffier D., Candresse T., Chatzivassiliou E., Dehnen-Schmutz K., Gilioli G., Grégoire J.-C., Jaques Miret J.A., MacLeod A., et al. Updated Pest Categorisation of Xylella Fastidiosa. EFSA J. 2018;16:e05357. doi: 10.2903/j.efsa.2018.5357.
2. Alston J.M., Fuller K.B., Kaplan J.D., Tumber K.P. Assessing the Returns to R&D on Perennial Crops: The Costs and Benefits of Pierce's Disease Research in the California Winegrape Industry. Aust. J. Agric. Resour. Econ. 2015;59:95-115. doi: 10.1111/1467-8489.12045.
3. Tumber K.P., Alston J.M., Fuller K. Pierce's Disease Costs California $104 Million per Year. Calif. Agric. 2014;68 doi: 10.3733/ca.v068n01p20.
4. Schneider K., van der Werf W., Cendoya M., Mounts M., Navas-Cortés J.A., Vicent A., Lansink A.O. Impact of Xylella Fastidiosa Subspecies Pauca in European Olives. PNAS. 2020;117:9250-9259. doi: 10.1073/pnas.1912206117.
5. Ali B.M., van der Werf W., Oude Lansink A. Assessment of the Environmental Impacts of Xylella Fastidiosa Subsp. Pauca in Puglia. Crop Prot. 2021;142:105519. doi: 10.1016/j.cropro.2020.105519.
6. Simpson A.J., Reinach F.C., Arruda P., Abreu F.A., Acencio M., Alvarenga R., Alves L.M., Araya J.E., Baia G.S., Baptista C.S., et al. The Genome Sequence of the Plant Pathogen Xylella Fastidiosa. The Xylella Fastidiosa Consortium of the Organization for Nucleotide Sequencing and Analysis. Nature. 2000;406:151-159. doi: 10.1038/35018003.
7. Falahi Charkhabi N., Booher N.J., Peng Z., Wang L., Rahimian H., Shams-Bakhsh M., Liu Z., Liu S., White F.F., Bogdanove A.J. Complete Genome Sequencing and Targeted Mutagenesis Reveal Virulence Contributions of Tal2 and Tal4b of Xanthomonas Translucens Pv. Undulosa ICMP11055 in Bacterial Leaf Streak of Wheat. Front. Microbiol. 2017;8 doi: 10.3389/fmicb.2017.01488.
8. Pieretti I., Royer M., Barbe V., Carrere S., Koebnik R., Cociancich S., Couloux A., Darrasse A., Gouzy J., Jacques M.-A., et al. The Complete Genome Sequence of Xanthomonas Albilineans Provides New Insights into the Reductive Genome Evolution of the Xylem-Limited Xanthomonadaceae. BMC Genom. 2009;10:616. doi: 10.1186/1471-2164-10-616.
9. Gerlin L., Cottret L., Cesbron S., Taghouti G., Jacques M.-A., Genin S., Baroukh C. Genome-Scale Investigation of the Metabolic Determinants Generating Bacterial Fastidious Growth. mSystems. 2020;5 doi: 10.1128/mSystems.00698-19
10. Kyrkou I, Pusa T., Ellegaard-Jensen L., Sagot MF, Hestbjerg Hansen L., Pierce's Disease of Grapevines : A Review of Control Strategies and an Outline of an Epidemiiological Model, frontiers in Microbiology, 12 September 2028, doi: 10.3389/fmicb.2018.02141
11. Richard A. Redak, Alexander H. Purcell, Joao R.S. Lopes, Matthew J. Blua, Russell F. Mizell III, Peter C. Andersen, The biology of xylem fluid - feeding insect vectors of Xylella fastidiosa and their relation to disease Epidemiology, Annu. Rev. Entomol. 2004, doi : 10.1146/annurev.ento.49.061802.123403
12. Wells J.M., Raju B.C., Hung H.-Y, Weisburg W.G., Mandelco-Paul L., Brenner D.J. Xylella Fastidiosa Gen. Nov., Sp. Nov: Gram-Negative, Xylem-Limited, Fastidious Plant Bacteria Related to Xanthomonas Spp. Int. J. Syst. Evol. Microbiol. 1987;37:136-143. doi: 10.1099/00207713-37-2-136.
13. Mansfield J., Genin S., Magori S., Citovsky V., Sriariyanum M., Ronald P., Dow M., Verdier V., Beer S.V., Machado M.A., et al. Top 10 Plant Pathogenic Bacteria in Molecular Plant Pathology: Top 10 Plant Pathogenic Bacteria. Mol. Plant Pathol. 2012;13:614-629. doi: 10.1111/j.1364-3703.2012.00804.x.
14. Jacques M.-A., Arlat M., Boulanger A., Boureau T., Carrère S., Cesbron S., Chen N.W.G., Cociancich S., Darrasse A., Denancé N., et al. Using Ecology, Physiology, and Genomics to Understand Host Specificity in Xanthomonas. Annu. Rev. Phytopathol. 2016;54:163-187. doi: 10.1146/annurev-phyto-080615-100147.
15. Bragard C., Dehnen-Schmutz K., Serio F.D., Gonthier P., Jacques M.-A., Miret J.A.J., Justesen A.F., MacLeod A., Magnusson C.S., Milonas P., et al. Effectiveness of in Planta Control Measures for Xylella Fastidiosa. EFSA J. 2019;17:e05666. doi: 10.2903/j.efsa.2019.5666.
16. Dupas E., Briand M., Jacques M.-A., Cesbron S. Novel Tetraplex Quantitative PCRAssays for Simultaneous Detection and Identification of Xylella Fastidiosa Subspecies in Plant Tissues. Frontiers in Plant Science. 2019. doi: 10.3389/fpls.2019.01732.
17. Directive 2006/7/EC of the European Parliament and of the Council of 15 February 2006 Concerning the Management of Bathing Water Quality and Repealing Directive 76/160/EEC. Off. J. Eur. Union. 2006;L64:37-51.
18. Andrea Luvisi; Francesca Nicoli; Luigi De Bellis Sustainable Management of Plant Quarantine Pests: The Case of Olive Quick Decline Syndrome. Sustainability. 2017;9:659. doi: 10.3390/su9040659.
19. EFSA Panel on Plant Health (PLH) Treatment Solutions to Cure Xylella Fastidiosa Diseased Plants. EFSA J. 2016;14 doi: 10.2903/j.efsa.2016.4456.
20. Scortichini M., Chen J., Caroli M.D., Dalessandro G., Pucci N., Modesti V., L'aurora A., Petriccione M., Zampella L., Mastrobuoni F., et al. A Zinc, Copper and Citric Acid Biocomplex Shows Promise for Control of Xylella Fastidiosa Subsp. Pauca in Olive Trees in Apulia Region (Southern Italy) Phytopathol. Mediterr. 2018;57:48-72. doi: 10.14601/Phytopathol_Mediterr-21985.
21. Hopkins D.L. Biological Control of Pierce's Disease in the Vineyard with Strains of Xylella Fastidiosa Benign to Grapevine. Plant Dis. 2005;89:1348-1352. doi: 10.1094/PD-89-1348.
22. Baccari C., Antonova E., Lindow S. Biological Control of Pierce's Disease of Grape by an Endophytic Bacterium. Phytopathology. 2019;:248-256. doi: 10.1094/PHYTO-07-18-0245-FI.
23. Lisa O'Sullivan, Colin Buttimer, Olivia McAuliffe, Declan Bolton, Aidan Coffey, Bacteriophage-based tools : recent advances and novel applications, 29 March 2022, F1000Research
24. Manyi-Loh C., Mamphweli S., Meyer E., Okoh A. Antibiotic Use in Agriculture and Its Consequential Resistance in Environmental Sources: Potential Public Health Implications. Molecules. 2018;23:795. doi: 10.3390/molecules23040795.
25. Zaczek M., Weber-D 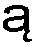 browska B., Górski A. Phages in the Global Fruit and Vegetable Industry. *J. Appl. Microbiol.* 2015;118:537-556. doi: 10.1111/jam.12700.
26. Sieiro C., Areal-Hermida L., Pichardo-Gallardo Á., Almuiña-González R., de Miguel T., Sánchez S., Sánchez-Pérez Á., Villa T.G. A Hundred Years of Bacteriophages: Can Phages Replace Antibiotics in Agriculture and Aquaculture? Antibiotics. 2020;9:493. doi: 10.3390/antibiotics9080493.
27. Buttimer C., McAuliffe O., Ross R.P., Hill C., O'Mahony J., Coffey A. Bacteriophages and Bacterial Plant Diseases. Front. Microbiol. 2017;8 doi: 10.3389/fmicb.2017.00034.
28. Ahern S.J., Das M., Bhowmick T.S., Young R., Gonzalez C.F. Characterization of Novel Virulent Broad-Host-Range Phages of Xylella Fastidiosa and Xanthomonas. J. Bacteriol. 2014;196:459-471. doi: 10.1128/JB.01080-13.
29. Das M., Bhowmick T.S., Ahern S.J., Young R., Gonzalez C.F. Control of Pierce's Disease by Phage. PLoS ONE. 2015;10:e0128902. doi: 10.1371/journal.pone.0128902.
30. Gonzalez C.F., Ahern S.J., Das M., Young I.R.F., Bhowmick T.S. Methods and Compositions for Treatment and Control of Plant Disease. No. 10,499,650. U.S. Patent. 2015 Dec 10.
31. Clavijo-Coppens F., Ginet N., Cesbron S., Briand M., Jacques M-A, Ansaldi M., Novel Virulent Bacteriophages Infecting Mediterranean Isolates of the Plant Pest Xylella fastidiosa and Xanthomonas albilineans, Viruses. 2021 Apr 21;13(5):725. doi: 10.3390/v13050725.
32. Bin Jang, H., Bolduc, B., Zablocki, O., Kuhn, J. H., Roux, S., Adriaenssens, E. M., Brister, J. R., Kropinski, A. M., Krupovic, M., Lavigne, R., Turner, D., & Sullivan, M. B. (2019). Taxonomic assignment of uncultivated prokaryotic virus genomes is enabled by gene-sharing networks. Nature Biotechnology, 37(6), 632-639. https://doi.org/10.1038/s41587-019-0100-8.
33. Davis, M. J., French, W. J., & Schaadw, N. W. (1981). Axenic Culture of the Bacteria Associated with Phony Disease of Peach and Plum Leaf Scald. In CURRENT MICROBIOLOGY (Vol. 6).
34. Farris, J. S. (n.d.). ESTIMATING PHYLOGENETIC TREES FROM DISTANCE MATRICES*».*
35. Fischer-Le Saux, M., Bonneau, S., Essakhi, S., Manceau, C., & Jacques, M. A. (2015). Aggressive emerging pathovars of Xanthomonas arboricola represent widespread epidemic clones distinct from poorly pathogenic strains, as revealed by multilocus sequence typing. Applied and Environmental Microbiology, 81(14), 4651-4668. https://doi.org/10.1128/AEM.00050-15
36. Gendre, J., Ansaldi, M., Olivenza, D. R., Denis, Y., Casadesús, J., & Ginet, N. (2022). Genetic Mining of Newly Isolated Salmophages for Phage Therapy. International Journal of Molecular Sciences, 23(16). https://doi.org/10.3390/ijms23168917
37. Göker, M., García-Blázquez, G., Voglmayr, H., Telleria, M. T., & Martin, M. P. (2009). Molecular taxonomy of phytopathogenic fungi: A case study in Peronospora. PLoS ONE, 4(7). https://doi.org/10.1371/journal.pone.0006319
38. Kropinski, A. M., Mazzocco, A., Waddell, T. E., Lingohr, E., & Johnson, R. P. (2009). Enumeration of Bacteriophages by Double Agar OverlayPlaque Assay (M. R. J. Clokie & A. M. Kropinski, Eds.; Vol. 501). Humana Press. https://doi.org/10.1007/978-1-60327-164-6.
39. Lefort, V., Desper, R., & Gascuel, O. (2015). FastME 2.0: A comprehensive, accurate, and fast distance-based phylogeny inference program. Molecular Biology and Evolution, 32(10), 2798-2800. https://doi.org/10.1093/molbev/msv150
40. Meier-Kolthoff, J. P., Auch, A. F., Klenk, H.-P., &·· Oker, M. G. (2013). Genome sequence-based species delimitation with confidence intervals and improved distance functions. In BMC Bioinformatics (Vol. 14). http://www.biomedcentral.com/1471-2105/14/60
41. Meier-Kolthoff, J. P., & Göker, M. (2017). VICTOR: genome-based phylogeny and classification of prokaryotic viruses. Bioinformatics (Oxford, England), 33(21), 3396-3404. https://doi.org/10.1093/bioinformatics/btx440
42. Meier-Kolthoff, J. P., Hahnke, R. L., Petersen, J., Scheuner, C., Michael, V., Fiebig, A., Rohde, C., Rohde, M., Fartmann, B., Goodwin, L. A., Chertkov, O., Reddy, T., Pati, A., Ivanova, N. N., Markowitz, V., Kyrpides, N. C., Woyke, T., Göker, M., & Klenk, H.-P. (2014). EXTEN D E D G E N Complete genome sequence of DSM 30083 T, the type strain (U5/41 T) of Escherichia coli, and a proposal for delineating subspecies in microbial taxonomy. http://www.standardsingenomics.com/content/9/ 1/2
43. Moraru, C., Varsani, A., & Kropinski, A. M. (2020). VIRIDIC-A novel tool to calculate the intergenomic similarities of prokaryote-infecting viruses. Viruses, 12(11). https://doi.org/10.3390/v12111268
44. Olm, M. R., Brown, C. T., Brooks, B., & Banfield, J. F. (2017). DRep: A tool for fast and accurate genomic comparisons that enables improved genome recovery from metagenomes through de-replication. ISME Journal, 11(12), 2864-2868. https://doi.org/10.1038/ismej.2017.126
45. Pandolfo, M., Telatin, A., Lazzari, G., Adriaenssens, E. M., & Vitulo, N. (2022). MetaPhage: an Automated Pipeline for Analyzing, Annotating, and Classifying Bacteriophages in Metagenomics Sequencing Data. MSystems, 7(5). https://doi.org/10.1128/msystems.00741-22
46. Schneider, C. A., Rasband, W. S., & Eliceiri, K. W. (2012). NIH Image to imaged: 25 years of Image Analysis HHS Public Access. In Nat Methods (Vol. 9, Issue 7).
47. Turner, D., Kropinski, A. M., & Adriaenssens, E. M. (2021). A roadmap for genome-based phage taxonomy. Viruses, 13(3). https://doi.org/10.3390/v13030506
48. Yu, G. (2020). Using ggtree to Visualize Data on Tree-Like Structures. Current Protocols in Bioinformatics, 69(1). https://doi.org/10.1002/cpbi.96
49. Korniienko, N., Kharina, A., Zrelovs, N., Jind 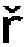 ichová, B., Moravec, T., Budzanivska, I., Burketová, L., & Kalachova, T. (2022). Isolation and Characterization of Two Lytic Phages Efficient Against Phytopathogenic Bacteria From Pseudomonas and Xanthomonas Genera. *Frontiers in Microbiology,* 13. https://doi.org/10.3389/fmicb.2022.853593
50. Taslem Mourosi, J., Awe, A., Guo, W., Batra, H., Ganesh, H., Wu, X., & Zhu, J. (2022). Understanding Bacteriophage Tail Fiber Interaction with Host Surface Receptor: The Key "Blueprint" for Reprogramming Phage Host Range. In International Journal of Molecular Sciences (Vol. 23, Issue 20). MDPI. https://doi.org/10.3390/ijms232012146
51. Campanharo, J. C., Lemos, M. V. F., & De Macedo Lemos, E. G. (2003). Growth optimization procedures for the phytopathogen Xylella fastidiosa. Current Microbiology, 46(2), 99-102. https://doi.org/10.1007/s00284-002-3829-z
52. Dann Turner et al., Abolishment of morphology-based taxa and change to binomial species names: 2022 taxonomy update of the ICTV bacterial viruses subcommittee DOI: 10.1007/s00705-022-05694-2

## Claims

1. An isolated bacteriophage that is aimed at *Xylella fastidiosa,* wherein the bacteriophage:
(a) is capable of inhibiting the growth of said *Xylella fastidiosa;*
(b) comprises a flexible non-contractile tail with a length > 220 nm and an icosahedral capsid
(c) exhibits a genomic size of about 55000 bp to 55500 bp; and
(d) comprises a genome with a DNA sequence having at least 92% sequence identity to SEQ ID NO:1.

2. The isolated bacteriophage according to claim 1, which comprises a genome with a DNA sequence selected in the group consisting of SEQ ID NO:1, SEQ ID NO:2 and SEQ ID NO:3.

3. The isolated bacteriophage according to claim 1 or 2, selected in the group consisting of the bacteriophages Aoi, Oroshi and Alme deposited respectively under the following DSMZ Accession Numbers : DSM 34782, DSM 34799 and DSM 34795.

4. A method of preventing or reducing symptoms or disease caused by *Xylella fastidiosa* in a plant, comprising contacting said plant or a part of said plant with at least one bacteriophage according to any one of claims 1 to 3.

5. The method according to claim 4, wherein contacting comprises introducing bacteriophage particles into the plant.

6. The method according to claim 4 or 5, wherein the plant is a grapevine, olive tree, citrus tree, almond tree, coffee tree, blackberry tree, mulberry tree, maple tree, walnut tree, date tree, pistachio tree, plane tree, plum tree, pear tree, oak tree.

7. The method according to any one of claims 4 to 6, wherein the number of said bacteriophage(s) introduced into said plant is from 1.10⁵ to 1.10¹⁰ PFU/ml.

8. The method according to any one of claims 4 to 7, wherein at least two bacteriophages according to any one of claims 1 to 3 are simultaneously or sequentially used.

9. A biocontrol composition comprising at least one bacteriophage according to any one of claims 1 to 3 and a carrier.

10. A biocontrol composition according to claim 9 comprising wherein at least two bacteriophages according to any one of claims 1 to 3.
